# EUROPEAN PATENT APPLICATION

(11) **EP 3 054 309 A1**
(43) Date of publication of application: **10.08.2016**
(21) Application number: 16154875.5
(22) Date of filing: 09.02.2016
(51) Int. Cl.: G01R 33/56

(54) **METHOD AND APPARATUS FOR PROCESSING MULTIPLE TIME SERIES OF MAGNETIC RESONANCE IMAGES**

(30) Priority: 09.02.2015 KR 20150019659
(71) Applicant: Samsung Electronics Co., Ltd., Gyeonggi-do 16677 (KR)
(72) Inventor: JO, Hyun-hee, Gyeonggi-do (KR); SHIN, Je-yong, Gyeonggi-do (KR); GULAKA, Praveen, Gyeonggi-do (KR)
(74) Representative: Land, Addick Adrianus Gosling

(57) **Abstract**

A method and an apparatus for processing a magnetic resonance (MR) image of an object, are provided. The method includes receiving a user input for displaying, on a screen, first MR images and second MR images that are obtained by performing an MR imaging (MRI) scan on the object at different times, classifying the first MR images into a plurality of first series according to a protocol of the object, and displaying the plurality of first series in a first region of the screen. The method further includes classifying the second MR images into a plurality of second series respectively corresponding to the plurality of first series, the plurality of second series being arranged in a second order that is same as a first order of the plurality of first series, and displaying the plurality of second series in a second region of the screen.

## Description

This application claims priority from Korean Patent Application No. 10-2015-0019659, filed on February 9, 2015, in the Korean Intellectual Property Office, the disclosure of which is incorporated herein by reference in its entirety.

Methods and apparatuses consistent with exemplary embodiments relate to methods and apparatuses for processing a magnetic resonance (MR) image.

A magnetic resonance imaging (MRI) apparatus uses a magnetic field to capture an image of a subject, and is widely used in the accurate diagnosis of diseases because it shows stereoscopic images of bones, lumbar discs, joints, nerve ligaments, the heart, etc., at selected angles. For example, an MRI apparatus may determine the presence of heart disease over time by obtaining MR images of the heart at predetermined time intervals for analysis.

A user of an MRI apparatus (hereinafter, referred to as an operator, radiologist, or manipulator) may acquire an image by manipulating the MRI apparatus. Because the user may repeatedly operate the MRI apparatus over a period of several years, convenient manipulation of the MRI apparatus is an issue of great concern. For example, when the user analyzes MRI images captured at different times, matching and comparing corresponding data is a cumbersome and time-consuming process.

Exemplary embodiments address at least the above problems and/or disadvantages and other disadvantages not described above. Also, the exemplary embodiments are not required to overcome the disadvantages described above, and may not overcome any of the problems described above.

One or more exemplary embodiments provide methods and apparatuses for processing a magnetic resonance (MR) image and for further facilitating image manipulation by a user.

One or more exemplary embodiments provide methods and apparatuses for processing an MR image and providing a user interface for facilitating comparison between two or more MR images captured at different times by arranging the two or more MR images so that they correspond to each other.

According to an aspect of an exemplary embodiment, there is provided a method of processing a magnetic resonance (MR) image of an object, the method including receiving a user input for displaying, on a screen, first MR images and second MR images that are obtained by performing an MR imaging (MRI) scan on the object at different times, classifying the first MR images into a plurality of first series according to a protocol of the object, and displaying the plurality of first series in a first region of the screen. The method further includes classifying the second MR images into a plurality of second series respectively corresponding to the plurality of first series, the plurality of second series being arranged in a second order that is same as a first order of the plurality of first series, and displaying the plurality of second series in a second region of the screen.

The receiving may include receiving a user input for displaying the first MR images on the screen, and receiving a user input for displaying the second MR images on the screen in response to the receiving the user input for displaying the first MR images.

The classifying the first MR images may include arranging the plurality of first series in a matrix form, and the classifying the second MR images may include arranging the plurality of second series in a matrix form.

The first MR images and the second MR images may be obtained by respectively performing MRI scans on different objects.

The method may further include receiving a user input for selecting a first series from the displayed plurality of first series, selecting the first series from the plurality of first series based on the user input for selecting the first series, and determining whether a second series corresponding to the selected first series is included in the plurality of second series.

The method may further include selecting the second series from the plurality of second series in response to the determining that the second series corresponding to the selected first series is included in the plurality of second series, and displaying an enlarged version of the selected second series in the second region.

The method may further include displaying a blank image in an area of the second region corresponding to the selected first series in response to the determining that the second series corresponding to the selected first series is not included in the plurality of second series.

The method may further include receiving a user input for changing the first order in which the plurality of first series is arranged, changing the first order in which the plurality of first series is arranged, based on the user input for changing the order in which the plurality of first series is arranged, and changing the second order in which the plurality of second series is arranged to correspond to the first order in which the plurality of first series is arranged.

The method may further include receiving a user input for searching for a first series among the plurality of first series and a second series among the plurality of second series, searching for the first series among the plurality of first series and the second series among the plurality of second series based on the user input for searching for the first series and the second series, displaying the first series found among the plurality of first series in the first region, and displaying the second series found among the plurality of second series in the second region.

Only the found first series and the found second series may be displayed on the screen.

The method may further include displaying, in a third region of the screen, one or more icons for executing one or more applications capable of analyzing the object.

The method may further include receiving a user input for selecting a first series from the displayed plurality of first series, displaying, on the selected first series, a mark distinguishing the selected first series from series unselected from the plurality of first series, and displaying, on an icon for executing an application capable of analyzing the object by using the selected first series, among the one or more icons, a mark identical to the mark displayed on the selected first series.

The method may further include selecting a second series corresponding to the selected first series from the plurality of second series, and displaying, on the selected second series, a mark identical to the mark displayed on the selected first series.

The method may further include receiving a user input for selecting a first series from the displayed plurality of first series, and displaying first MR images included in the selected first series sequentially with respect to time.

The method may further include selecting a second series corresponding to the selected first series from the plurality of second series, and displaying second MR images included in the selected second series sequentially with respect to time.

The method may further include receiving a user input for displaying, on the screen, third MR images that are obtained by performing an MRI scan on the object, classifying the third MR images into a plurality of third series respectively corresponding to the plurality of first series and the plurality of second series, the plurality of third series being arranged in a third order that is same as the first order of the plurality of first series and the second order of the plurality of second series, and the plurality of third series being arranged in a matrix form, and displaying the plurality of third series in a third region of the screen.

A non-transitory computer-readable storage medium may store a program including instructions to cause a computer to perform the method.

According to an aspect of another exemplary embodiment, there is provided an apparatus for processing an MR image of an object, the apparatus including an image processor configured to receive a user input for displaying, on a screen, first MR images and second MR images that are obtained by performing an MRI scan on the object at different times, classify the first MR images into a plurality of first series according to a protocol of the object, and classify the second MR images into a plurality of second series respectively corresponding to the plurality of first series, the plurality of second series being arranged in a second order that is same as a first order of the plurality of first series. The apparatus further includes a display including the screen including a first region and a second region, the display being configured to display the plurality of first series in the first region, and display the plurality of second series in the second region.

The image processor may be further configured to arrange the plurality of first series in a matrix form, and arrange the plurality of second series in a matrix form.

The image processor may include a user input interface configured to receive the user input for displaying the first MR images and the second MR images.

The user input interface may be further configured to receive a user input for selecting a first series from the displayed plurality of first series, and the image processor may be further configured to select the first series from the plurality of first series based on the user input for selecting the first series, and determine whether a second series corresponding to the selected first series is included in the plurality of second series.

The image processor may be further configured to select the second series from the plurality of second series in response to the image processor determining that the second series corresponding to the selected first series is included in the plurality of second series, and the display may be further configured to display an enlarged version of the selected first series in the first region, and display an enlarged version of the selected second series in the second region.

The display may be further configured to display a blank image in an area of the second region corresponding to the selected first series in response to the image processor determining that the second series corresponding to the selected first series is not included in the plurality of second series.

The user input interface may be further configured to receive a user input for changing the first order in which the plurality of first series is arranged, and the image processor may be further configured to change the first order in which the plurality of first series is arranged, based on the user input for changing the first order in which the plurality of first series is arranged, and change the second order in which the plurality of second series is arranged to correspond to the first order in which the plurality of first series is arranged.

The user input interface may be further configured to receive a user input for searching for a first series among the plurality of first series and a second series among the plurality of second series, and the image processor may be further configured to search for the first series among the plurality of first series and the second series among the plurality of second series based on the user input for searching for the first series and the second series.

The display may be further configured to display the first series found among the plurality of first series in the first region, and display the second series found among the plurality of second series in the second region.

Only the found first series and the found second series may be displayed on the screen.

The user input interface may be further configured to receive a user input for selecting a first series from the displayed plurality of first series, and the display may be further configured to display, on the screen, an icon for executing an application capable of analyzing the object by using the selected first series based on the user input for selecting the first series.

The display may be further configured to display, in the first region, first analysis data for the object that is obtained by executing the application.

The image processor may be further configured to select the second series corresponding to the selected first series from the plurality of second series, and the display may be further configured to display, in the second region, second analysis data for the object that is obtained by executing the application using the selected second series.

The display may be further configured to display, on the selected first series, a mark distinguishing the selected first series from series unselected from the plurality of first series, and display, on the icon for executing the application capable of analyzing the object by using the selected first series, a mark identical to the mark displayed on the selected first series.

The user input interface may be further configured to receive a user input for selecting a first series from the displayed plurality of first series, and the display may be further configured to display first MR images included in the selected first series sequentially with respect to time.

The image processor may be further configured to select a second series corresponding to the selected first series from the plurality of second series, and the display may be further configured to display second MR images included in the selected second series sequentially with respect to time.

The protocol may be of obtaining the first MR images.

### BRIEF DESCRIPTION OF THE DRAWINGS

The above and/or other aspects will be more apparent by describing exemplary embodiments, with reference to the accompanying drawings, in which:
FIG. 1 is a diagram of a magnetic resonance imaging (MRI) apparatus according to an exemplary embodiment;
FIG. 2 is a diagram for explaining a method of arranging MR images according to an exemplary embodiment;
FIG. 3 is a flowchart of a method of arranging MR images according to an exemplary embodiment;
FIG. 4 is a diagram for explaining a method of arranging MR images according to an exemplary embodiment;
FIG. 5 is a flowchart of a method of arranging MR images according to an exemplary embodiment;
FIG. 6 is a diagram for explaining a method of processing an MR image when a user selects the MR image, according to an exemplary embodiment;
FIG. 7 is a flowchart of a method of processing an MR image when a user selects the MR image, according to an exemplary embodiment;
FIG. 8 is a diagram for explaining a method of processing an MR image when a user selects the MR image, according to an exemplary embodiment;
FIG. 9 is a flowchart of a method of processing an MR image when a user selects the MR image, according to an exemplary embodiment;
FIG. 10 is a diagram for explaining a method of displaying an MR image according to an exemplary embodiment;
FIG. 11 is a flowchart of a method of displaying an MR image according to an exemplary embodiment;
FIG. 12 is a diagram for explaining a method of changing an order in which MR images are arranged, according to an exemplary embodiment;
FIG. 13 is a flowchart of a method of changing an order in which MR images are arranged, according to an exemplary embodiment;
FIG. 14 is a diagram for explaining a method of searching for an MR image according to an exemplary embodiment;
FIG. 15 is a flowchart of a method of searching for an MR image according to an exemplary embodiment;
FIG. 16 is a diagram of a search region in an apparatus for processing an MR image, and an enlarged version of the MR image, according to an exemplary embodiment;
FIG. 17 is a diagram for explaining a method of executing an application using an MR image according to an exemplary embodiment;
FIG. 18 is a diagram for explaining a method of displaying analysis data for an object obtained by executing an MR image processing application, according to an exemplary embodiment;
FIG. 19 is a flowchart of a method of executing an MR image processing application according to an exemplary embodiment;
FIG. 20 is a diagram for explaining a method of executing an application using an MR image according to an exemplary embodiment;
FIG. 21 is a diagram for explaining a method of displaying analysis data for an object obtained by executing an MR image processing application, according to an exemplary embodiment;
FIG. 22 is a diagram for explaining a method of sequentially displaying MR images according to an exemplary embodiment;
FIG. 23 is a flowchart of a method of sequentially displaying MR images according to an exemplary embodiment;
FIG. 24 is a diagram for explaining a method of sequentially displaying MR images according to an exemplary embodiment;
FIG. 25 is a flowchart of a method of sequentially displaying MR images according to an exemplary embodiment; and
FIG. 26 is a schematic diagram of an MRI system.

### DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions may not be described in detail because they would obscure the description with unnecessary detail.

It will be understood that the terms "comprises" and/or "comprising" used herein specify the presence of stated features or components, but do not preclude the presence or addition of one or more other features or components. In addition, the terms such as "unit", "-er (-or)", and "module" described in the specification refer to an element for performing at least one function or operation, and may be implemented in hardware, software, or the combination of hardware and software.

Expressions such as "at least one of," when preceding a list of elements, modify the entire list of elements and do not modify the individual elements of the list.

In the present specification, an "image" may refer to multi-dimensional data composed of discrete image elements (e.g., pixels in a two-dimensional (2D) image and voxels in a three-dimensional (3D) image). For example, the image may be a medical image of an object captured by an X-ray apparatus, a computed tomography (CT) apparatus, a magnetic resonance imaging (MRI) apparatus, an ultrasound diagnosis apparatus, or another medical imaging apparatus.

Furthermore, in the present specification, an "object" may be a human, an animal, or a part of a human or animal. For example, the object may be an organ (e.g., the liver, the heart, the womb, the brain, a breast, or the abdomen), a blood vessel, or a combination thereof. Furthermore, the "object" may be a phantom. The phantom refers to a material having a density, an effective atomic number, and a volume that are approximately the same as those of an organism. For example, the phantom may be a spherical phantom having properties similar to the human body.

Furthermore, in the present specification, a "user" may be, but is not limited to, a medical expert, such as a medical doctor, a nurse, a medical laboratory technologist, or a technician who repairs a medical apparatus.

Furthermore, in the present specification, an "MR image" refers to an image of an object obtained by using the nuclear magnetic resonance principle.

Furthermore, in the present specification, a "pulse sequence" refers to continuity of signals repeatedly applied by an MRI apparatus. The pulse sequence may include a time parameter of a radio frequency (RF) pulse, for example, repetition time (TR) or echo time (TE).

Furthermore, in the present specification, a "pulse sequence schematic diagram" shows an order of events that occur in an MRI apparatus. For example, the pulse sequence schematic diagram may be a diagram showing an RF pulse, a gradient magnetic field, an MR signal, or the like according to time.

An MRI system is an apparatus for acquiring a sectional image of a part of an object by expressing, in a contrast comparison, a strength of a MR signal with respect to a radio frequency (RF) signal generated in a magnetic field having a strength. For example, if an RF signal that only resonates an atomic nucleus (for example, a hydrogen atomic nucleus) is emitted for an instant toward the object placed in a strong magnetic field and then such emission stops, an MR signal is emitted from the atomic nucleus, and thus the MRI system may receive the MR signal and acquire an MR image. The MR signal denotes an RF signal emitted from the object. An intensity of the MR signal may be determined according to a density of a predetermined atom (for example, hydrogen) of the object, a relaxation time T1, a relaxation time T2, and a flow of blood or the like.

MRI systems include characteristics different from those of other imaging apparatuses. Unlike imaging apparatuses such as CT apparatuses that acquire images according to a direction of detection hardware, MRI systems may acquire 2D images or 3D volume images that are oriented toward an optional point. MRI systems do not expose objects or examiners to radiation, unlike CT apparatuses, X-ray apparatuses, position emission tomography (PET) apparatuses, and single photon emission CT (SPECT) apparatuses, may acquire images having high soft tissue contrast, and may acquire neurological images, intravascular images, musculoskeletal images, and oncologic images that are used to precisely capture abnormal tissues.

Furthermore, in the present specification, a "series" refers to a set of slices obtained by classifying images of an object according to a protocol. Each of the images to be classified may be acquired using the principle of nuclear magnetic resonance.

Furthermore, in the present specification, the terms "first", "second", "I-1", etc. are only used to distinguish one element or component from another element or component. Thus, these terms are not limited to representing the order or priority among elements or components.

Furthermore, in the present specification, a "blank image" may be a blank having a size and a shape corresponding to an MR image but containing no content therein.

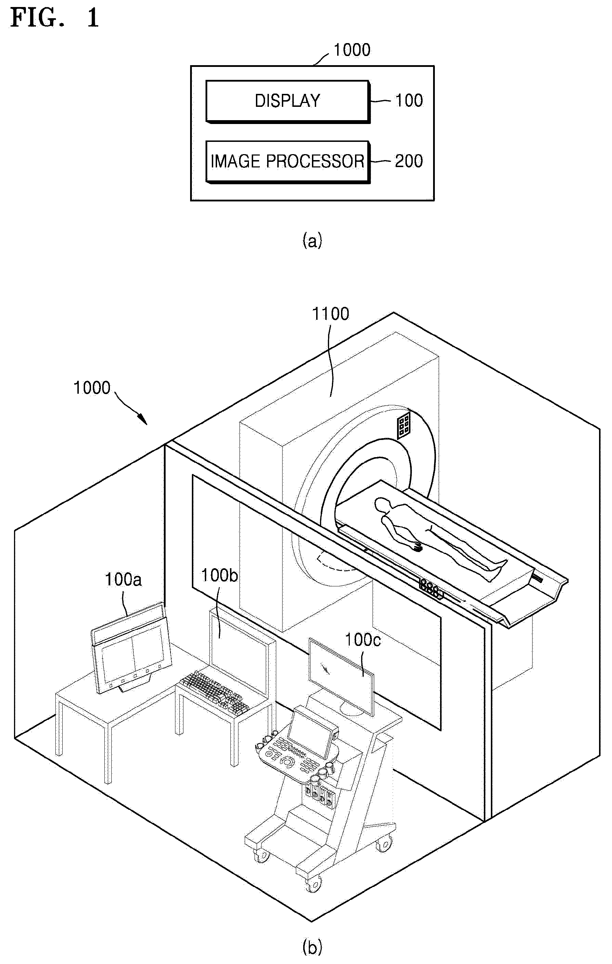

FIG. 1 is a diagram of an MRI apparatus 1000 according to an exemplary embodiment.

Referring to view (a) of FIG. 1, the MRI apparatus 1000 according to an exemplary embodiment includes a display 100 and an image processor 200.

The image processor 200 may process an MR signal received from an object to generate MR data of the object.

The image processor 200 receives the MR signal received by an RF receiver and performs any one of various signal processes, such as amplification, frequency transformation, phase detection, low frequency amplification, and filtering, on the received MR signal.

For example, the image processor 200 may arrange digital data in a k space (for example, also referred to as a Fourier space or a frequency space) of a memory, and rearrange the digital data into image data via 2D or 3D Fourier transformation.

The image processor 200 may perform a composition process or difference calculation process on rearranged image data. The composition process may include an addition process on a pixel or a maximum intensity projection (MIP) process. Furthermore, the image processor 200 may store not only the rearranged image data but also image data on which a composition process or a difference calculation process is performed, in a memory or an external server.

The image processor 200 may perform any of the signal processes on the MR signal in parallel. For example, the image processor 200 may perform a signal process on a plurality of MR signals received by a multi-channel RF coil in parallel to rearrange the MR signals into image data.

The display 100 may display an MR image generated or reconstructed by the image processor 200 to the user. The display 100 may also display a graphic user interface (GUI) as well as information used for the user to manipulate an MRI system, such as user information or object information. Examples of the display 100 may include a cathode ray tube (CRT) display, a liquid crystal display (LCD), a plasma display panel (PDP), an organic light emitting diode (OLED) display, a field emission display (FED), an LED display, a vacuum fluorescent display (VFD), a digital light processing (DLP) display, a flat panel display (FPD), a 3D display, a transparent display, etc.

View (b) of FIG. 1 illustrates an MRI apparatus 1000 according to an exemplary embodiment.

Referring to view (b) of FIG. 1, the MRI apparatus 1000 according to an exemplary embodiment includes a measurer 1100 and displays 100a, 100b, and 100c.

To generate an image of an object, the measurer 1100 may apply an MR signal to an object and receive an MR signal from the object. The measurer 1100 may include a gantry having a main magnet, a gradient coil, a radio frequency (RF) coil, etc. For example, the measurer 1100 may include the image processor 200 shown in view (b) of FIG. 1.

The displays 100a, 100b, and 100c may receive MR images generated by the measurer 1100 to display the MR images via various GUIs.

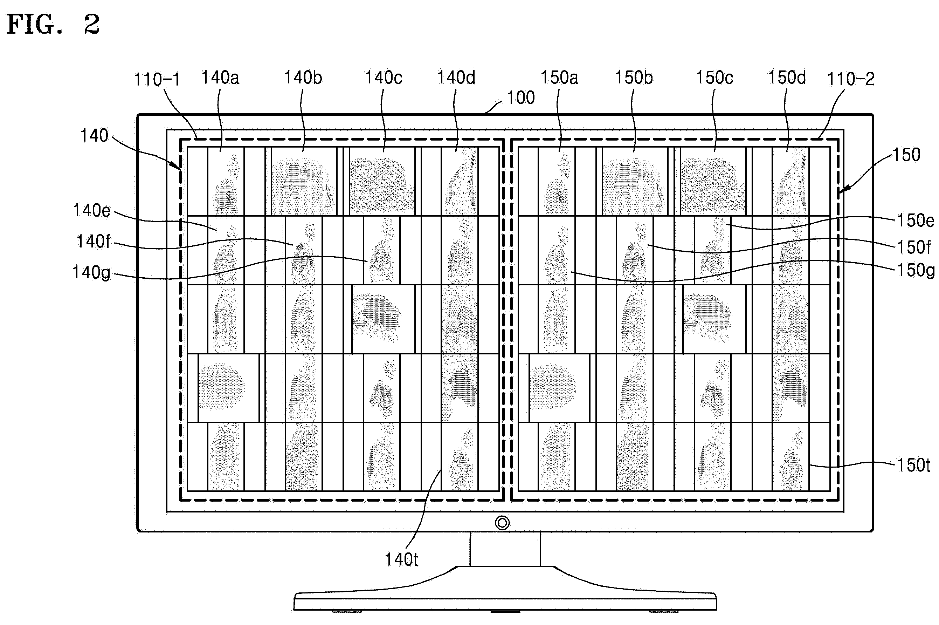

FIG. 2 is a diagram for explaining a method of arranging MR images according to an exemplary embodiment.

Referring to FIG. 2, the display 100 includes a screen that is partitioned into a first region 110-1 and a second region 110-2. A plurality of first MR images 140 are classified into a plurality of series 140a through 140t, and the series 140a through 140t arranged in matrix are displayed in the first region 110-1. A plurality of second MR images 150 are classified into a plurality of series 150a through 150t, and the series 150a through 150t arranged in matrix are displayed in the second region 110-2. Like the series 140a through 140t, each of the series 150a through 150t may include at least one MR image. While FIG. 2 shows that the first MR images 140 are classified into the series 140a through 140t, e.g., twenty (20) series arranged in a matrix having five (5) rows and four (4) columns, the number of the series 140a through 140t is not limited to 20. Similarly, although FIG. 2 also shows that the second MR images 150 are classified into the series 150a through 150t, i.e., the twenty (20) series having five (5) rows and four (4) columns of series, the number of the series 150a through 150t is not limited to 20.

Each of the series 140a through 140t obtained by classifying the first MR images 140 may be a set of slices into which MR images obtained by performing MRI of the object are divided according to each protocol. Each of the series 140a through 140t may include at least one MR image.

The second MR images 150 may be classified into the series 150a through 150t respectively corresponding to the series 140a through 140t of the first MR images 140. Furthermore, the series 150a through 150t may be arranged in the same order in which the corresponding series 140t through 140t are arranged. The series 140a through 140t may be arranged in the first region 110-1, the series 150a through 150t may be arranged in the second region 110-2, and matrix positions of the series 140a through 140t in the first region 110-1 may respectively correspond to matrix positions of the series 150a through 150t in the second region 110-2. Series arranged at corresponding matrix positions from among series 140a through 140t and the series 150a through 150t may include MR images of the object, which are acquired according to the same protocol. For example, the first series 140a located in the first region 110-1 may correspond to the first series 150a in the second region 110-2. Furthermore, the second series 140b located in the first region 110-1 may correspond to the second series 150b located in the second region 110-2. In the same manner, the twentieth series 140t in the first region 110-1 may correspond to the twentieth series 150t in the second region 110-2.

According to an exemplary embodiment, the first MR images 140 and the second MR images 150 respectively displayed in the first and second regions 110-1 and 110-2 may be images obtained by performing nuclear magnetic resonance on the same object at different times. For example, the first MR images 140 may be images acquired by scanning an object of a patient at a previous time point, and the second MR images 150 may be acquired by rescanning the same object of the patient at a current time point. Thus, according to an exemplary embodiment, previously and currently generated images of the same object may be respectively displayed parallel to one another in the first and second regions 110-1 and 110-2 of the screen of the display 100 and respectively classified and assigned to a plurality of series that are arranged such that each series in the previously generated images corresponds to a corresponding series in the currently generated images. This arrangement allows a user to easily compare data acquired from the same patient.

However, scanning time points and scanning orders for the first MR images 140 and the second MR images 150 are not limited to the above-described examples.

According to an exemplary embodiment, the first MR images 140 and the second MR images 150 respectively displayed in the first and second regions 110-1 and 110-2 may be obtained by performing nuclear magnetic resonance on different objects. For example, the first MR images and the second MR images may be acquired by respectively scanning objects of first and second patients.

The MRI apparatus 1000 according to an exemplary embodiment is configured to display the first MR images 140 and the second MR images 150 in the first and second regions 110-1 and 110-2 of the same screen, and the user may compare the first MR images 140 and the second MR images 150 side-by-side. For example, if the first MR images 140 and the second MR images 150 are obtained by performing MRI of the same object at different times, the user may be able to analyze progress of lesions in the patient, a state of the object, etc., and thereby easily analyze patient data.

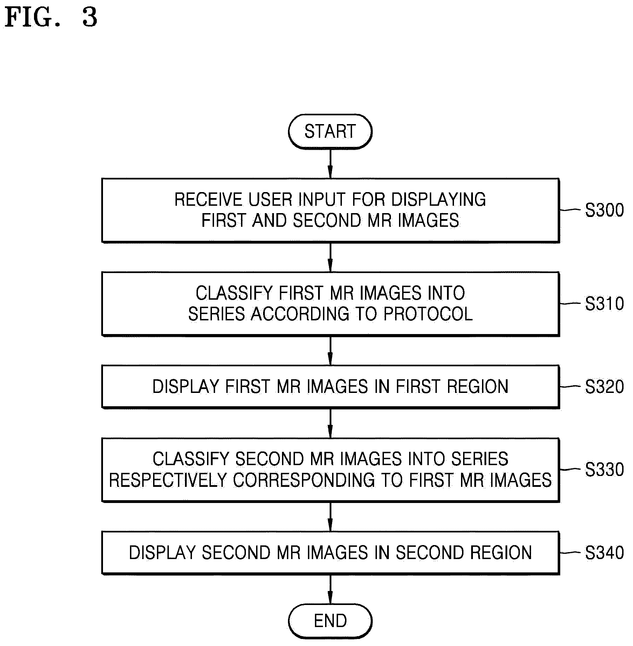

FIG. 3 is a flowchart of the method of arranging MR images illustrated in FIG. 2.

Referring to FIG. 3, the image processor 200 of view (a) of FIG. 1 receives a user input for displaying a plurality of first MR images and a plurality of second MR images on a screen of the display 100 of view (a) of FIG. 1 (S300). As described with reference to FIG. 2, the first MR images and the second MR images may be obtained by scanning an object of the same patient at different times.

In an exemplary embodiment, the image processor 200 may sequentially receive a user input for displaying the first MR images on the screen and a user input for displaying the second MR images on the screen. In detail, the image processor 200 may first receive a first user input for displaying the first MR images in the first region 110-1 of FIG. 2 and then a second user input for displaying the second MR images in the second region 110-2 of FIG. 2.

The image processor 200 classifies the first MR images into a plurality of series according to a protocol of an object (S310). According to an exemplary embodiment, as illustrated in FIG. 2, the first MR images may be classified into the series, i.e., the first through twentieth series 140a through 140t of FIG. 2. The image processor 200 may classify the first MR images into the series 140a through 140t and arrange the series 140a through 140t in a matrix.

The display 100 displays the first MR images in the first region 110-1 of the screen of the display 100 (S320). The display 100 may display the first MR images in the first region 110-1 according to the order in which the image processor 200 arranges the series 140a through 140t for the first MR images. According to an exemplary embodiment, the series 140a through 140t may be arranged and displayed in five rows and four columns.

The image processor 200 classifies the second MR images into the series 150a through 150t of FIG. 2 respectively corresponding to the series 140a through 140t obtained by classifying the first MR images (S330). The image processor 200 may arrange the series 150a through 150t in the same order in which the corresponding plurality of series 140a through 140t are arranged.

The display 100 displays the second MR images in the second region 110-2 of the screen (S340). The display 100 may display the second MR images in the second region 110-2 according to the order that the image processor 200 arranges the series 150a through 150t. The second region 110-2 may have the same area as the first region 110-1 where the first MR images are displayed.

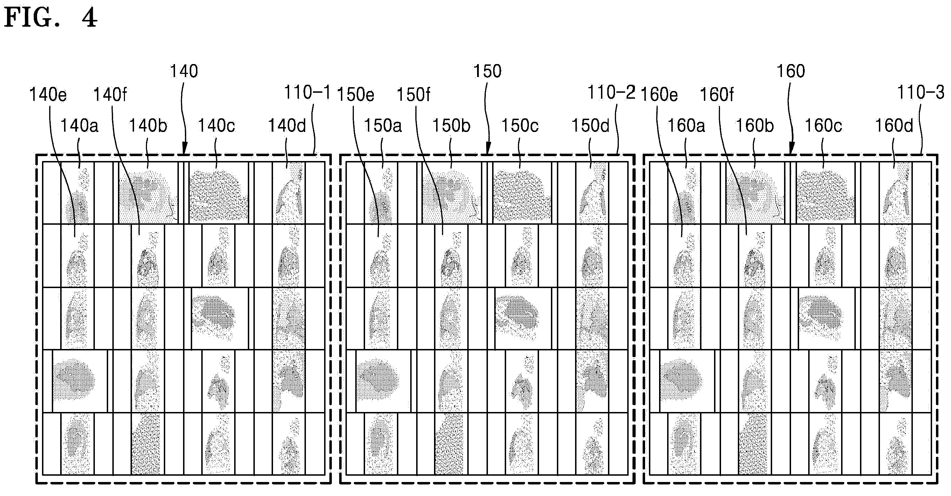

FIG. 4 is a diagram for explaining a method of arranging MR images according to an exemplary embodiment.

Referring to FIG. 4, the display 100 includes a screen that is partitioned into first through third regions 110-1 through 110-3. In an exemplary embodiment, the first through third regions 110-1 through 110-3 each have the same area. The plurality of first MR images 140 are classified into the plurality of series 140a through 140t, and the series 140a through 140t arranged in matrix are displayed in the first region 110-1. The plurality of second MR images 150 are classified into the plurality of series 150a through 150t, and the series 150a through 150t arranged in matrix are displayed in the second region 110-2. A plurality of third MR images 160 are classified into a plurality of series 160a through 160t, and the series 160a through 160t arranged in matrix are displayed in the third region 110-3.

As described with reference to FIG. 2, the first MR images 140 may be classified into the series 140a through 140t, each of which includes at least one MR image. Similarly, the second MR images 150 and the third MR images 160 may respectively be classified into the series 150a through 150t and the series 160a through 160t.

The second MR images 150 may be classified into the series 150a through 150t respectively corresponding to the series 140a through 140t. Furthermore, the second MR images 160 may be classified into the series 160a through 160t respectively corresponding to the series 150a through 150t. According to an exemplary embodiment, the first MR images 140, the second images 150, and the third MR images 160 may be respectively classified into the series 140a through 140t, 150a through 150t, and 160a through 160t corresponding to one another.

According to an exemplary embodiment, the series 150a through 150t obtained by classifying the second images 150 may be arranged in the same order in which the corresponding plurality of series 140a through 140t obtained by classifying the first images 140 are arranged. Similarly, the series 160a through 160t obtained by classifying the third images 160 may be arranged in the same order in which the corresponding plurality of series 150a through 150t are arranged. For example, the first MR images 140, the second MR images 150, and the third MR images 160 may respectively be classified into the series 140a through 140t, 150a through 150t, and 160a through 160t, and the series 140a through 140t, the series 150a through 150t, and the series 160a through 160t may respectively be arranged in the same order and displayed in the first through third regions 110-1 through 110-3. For example, the second series 160b displayed in the third region 110-3 may correspond to the second series 140b displayed in the first region 110-1. Furthermore, the fourth series 150d displayed in the second region 110-2 may correspond to the fourth series 160d displayed in the third region 110-3.

According to an exemplary embodiment, the first MR images 140, the second MR images 150, and the third MR images 160 respectively displayed in the first through third regions 110-1 through 110-3 may be images obtained by performing nuclear magnetic resonance on the same object at different times. For example, the first MR images 140 may be acquired by scanning an object of a patient at a first time point in the past, and the second MR images 150 may be acquired by rescanning the same object of the patient at a second time point that is later than the first time point. The third MR images may be acquired by rescanning the same object of the patient at a current time point that is later than the second time point. However, scanning time points and scanning orders for the first through third MR images 140, 150, and 160 are not limited thereto.

According to an exemplary embodiment, the first MR images 140, the second MR images 150, and the third MR images respectively displayed in the first through third regions 110-1 through 110-3 may be obtained by performing nuclear magnetic resonance on different objects.

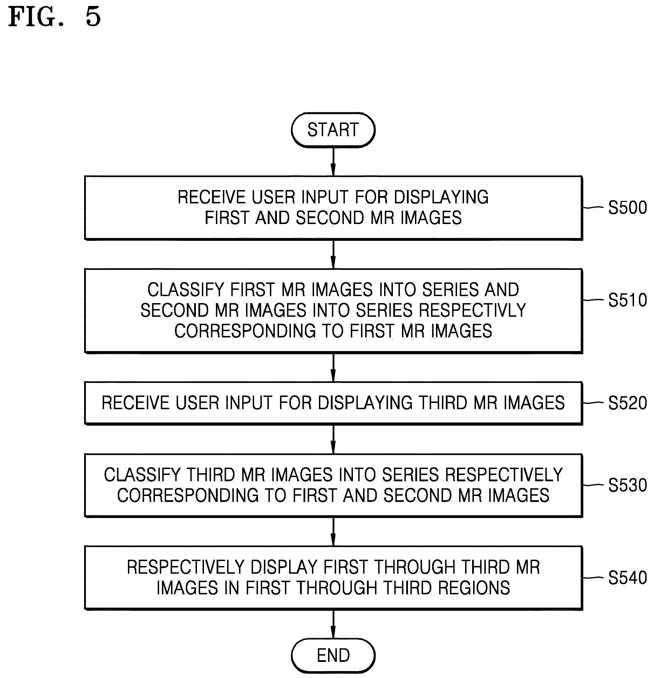

FIG. 5 is a flowchart of the method of arranging MR images illustrated in FIG. 4.

Referring to FIG. 5, the image processor 200 of view (a) of FIG. 1 receives a user input for displaying a plurality of first MR images and a plurality of second MR images on a screen of the display 100 of view (a) of FIG. 1 (S500). In an exemplary embodiment, the image processor 200 may include a user input interface. According to an exemplary embodiment, the image processor 200 may sequentially receive a user input for displaying the first MR images on the screen and a user input for displaying the second MR images on the screen.

The image processor 200 classifies the first MR images into a plurality of series according to a protocol of an object (S510). According to an exemplary embodiment, the first MR images may be classified into the series 140a through 140t of FIG. 4. The image processor 200 also classifies the second MR images into a plurality of series respectively corresponding to the series obtained by classifying the first MR images. In an exemplary embodiment, the image processor 200 may classify the second MR images into the series 150a through 150t. The image processor 200 may arrange the series 150a through 150t in the same order in which the corresponding plurality of series 140a through 140t are arranged.

The image processor 200 receives a user input for displaying a plurality of third images on the screen (S520). According to an exemplary embodiment, operations S500 and S520 may be performed simultaneously without a time delay. For example, the image processor 200 may receive a user input for displaying the first and second MR images on the screen simultaneously with a user input for displaying the third MR images on the screen.

The image processor 200 classifies the third MR images into a plurality of series respectively corresponding to the series for the first MR images and corresponding to the series for the second MR images (S530). According to an exemplary embodiment, the image processor 200 may classify the third MR images into the series 160a through 160t of FIG. 4 respectively corresponding to the series 140a through 140t and corresponding to the series 150a through 150t.

In an exemplary embodiment, the first MR images, the second MR images, and the third MR images may respectively be classified into the series corresponding to one another. For example, the second series 160b in the third MR images may correspond to the second series 140b for the first MR images, and the fourth series 150d in the second MR images may correspond to the fourth series 160d in the third MR images.

The display 100 displays the first MR images, the second MR images, and the third MR images in the first through third regions 110-1 through 110-3 of the screen, respectively (S540). The display 100 may display the first MR images, the second MR images, and the third MR images in the first through third regions 110-1 through 110-3 according to the order that the image processor 200 respectively arranges the series 140a through 140t, 150a through 150t, and 160a through 160t. In an exemplary embodiment, the through third regions 110-1 through 110-3 of the screen each may have the same area.

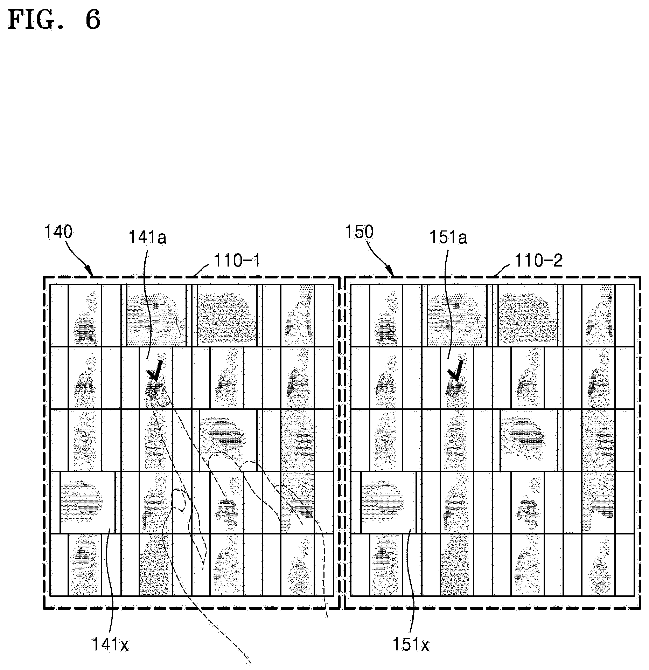

FIG. 6 is a diagram for explaining a method of processing an MR image when a user selects the MR image, according to an exemplary embodiment.

Referring to FIG. 6, the plurality of first MR images 140 are classified into a plurality of series and displayed in the first region 110-1 of a screen, and the plurality of second MR images 150 are classified into a plurality of series and displayed in the second region 110-2 of the screen. According to an exemplary embodiment, if a user selects one of the series displayed in the first region 110-1, a series corresponding to the selected series may be selected from among the series displayed in the second region 110-2 without a separate input for selecting a series from among the series in the second region 110-2. For example, if the user selects a series 1-1 141a arranged and displayed at position 1-1 in the first region 110-1, a series 2-1 151a at position 2-1 corresponding to the series 1-1 141a is selected from among the series in the second region 110-2 without an input for selecting a series from among the series in the second region 110-2.

According to an exemplary embodiment, if the user selects one of the series in the first region 110-1, a blank image may be selected when there is no series corresponding to the selected series in the second region 110-2. For example, if the user selects a series 1-2 141x at position 1-2 from among the series arranged and displayed in the first region 110-1, a blank image 151x is selected at a position in the second region 110-2 corresponding to the series 1-2 141x when there is no series corresponding to the series 1-2 141x in the second region 110-2.

The MRI apparatus 1000 according to an exemplary embodiment is configured so that, if an MR image is selected from among the first MR images 140 displayed in the first region 110-1, a series corresponding to the selected MR image is selected from among the second MR images 150 displayed in the second region 110-2. Thus, the time, cost, and effort for the user to manually search for corresponding MR images of the object may be reduced.

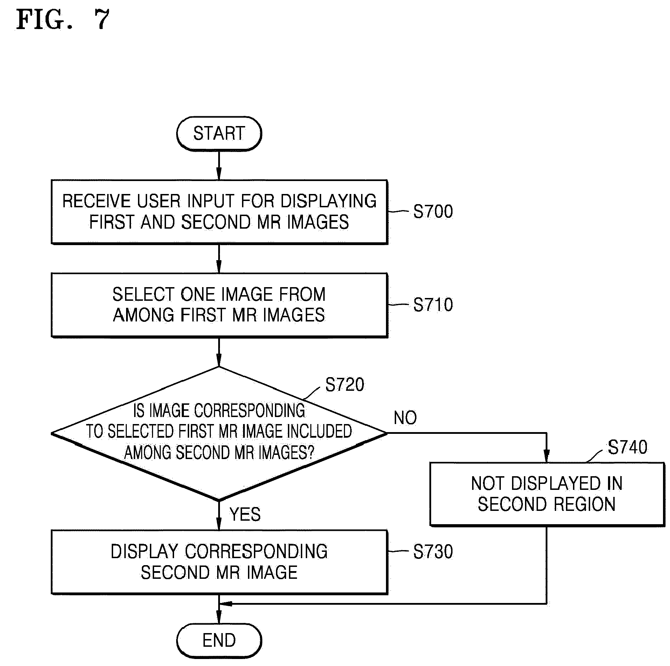

FIG. 7 is a flowchart of a method of processing an MR image when a user selects the MR image, according to an exemplary embodiment.

Referring to FIG. 7, the image processor 200 of view (a) of FIG. 1 receives a user input for displaying a plurality of first MR images and a plurality of second MR images on a screen of the display 100 of view (a) of FIG. 1 (S700). The image processor 200 may include a user input interface. As described with reference to FIG. 2, the first MR images and the second MR images may be MR images acquired by scanning an object of the same patient at different times.

In an exemplary embodiment, in operation S700, the image processor 200 may respectively classify the first MR images and the second MR images into a plurality of series according to protocols of an object, based on the received user input. Furthermore, the image processor 200 may classify the second MR images into a plurality of series respectively corresponding to the series obtained by classifying the first MR images.

The image processor 200 receives a user input for selecting one first MR image from among the first MR images (S710). According to an exemplary embodiment, in operation S700, the image processor 200 may respectively classify the first MR images and the second MR images into a plurality of series corresponding to one another, and receive a user input for selecting a series from among the series for the first MR images. For example, referring to FIG. 6 together, the image processor 200 may select a series 1-1 150a or series 1-2 141x arranged and displayed in the first region 110-1.

The image processor 200 determines whether the second MR images include an image corresponding to the selected one first MR image among the first MR images (S720). In an exemplary embodiment, the image processor 200 may determine whether a plurality of series for the second MR images include a series corresponding to a series in a plurality of series for the first MR images selected in operation S710. For example, referring back to FIG. 6 together, if the user selects the series 1-1 141a or series 1-2 141x, the image processor 200 may determine whether a series corresponding to the series 1-1 141a or the series 1-2 141x is included among the series for the second MR images arranged and displayed in the second region 110-2.

If the image processor 200 determines that the second MR images include an image corresponding to the selected one first MR image among the first MR images, the image processor 200 selects the second MR image corresponding thereto, and the display 100 displays the corresponding selected second MR image (S730). According to an exemplary embodiment, a series corresponding to a series in a plurality of series for the first MR images selected in operation S710 are included among a plurality of series for the second MR images, the image processor 200 may select the series corresponding thereto, and the display 100 may display the selected series on the screen. Referring to FIG. 6 together, if the image processor 200 receives a user input for selecting the series 1-1 141a, the image processor 200 may select a series 2-1 151a corresponding to the series 1-1 141a from among the second MR images arranged and displayed in the second region 110-2. Furthermore, the display 100 may display the series 2-1 151a in the second region 110-2.

On the other hand, if the image processor 200 determines that the second MR images does not include an image corresponding to the selected one first MR image among the first MR images, the display 100 displays no content at a position in the second region 110-2 of FIG. 6 corresponding to the selected first MR image (S740). According to an exemplary embodiment, if a series corresponding to a series in a plurality of series for the first MR images selected in operation S710 is not included among in a plurality of series for the second MR images, the display 100 may display a blank image in the second region 110-2. Referring to FIG. 6 together, if the image processor 200 receives a user input for selecting the series 1-2 141x, the display 100 may display a blank image 151x having no content at a position corresponding to the series 1-2 141x, among the series for the second MR images arranged and displayed in the second region 110-2.

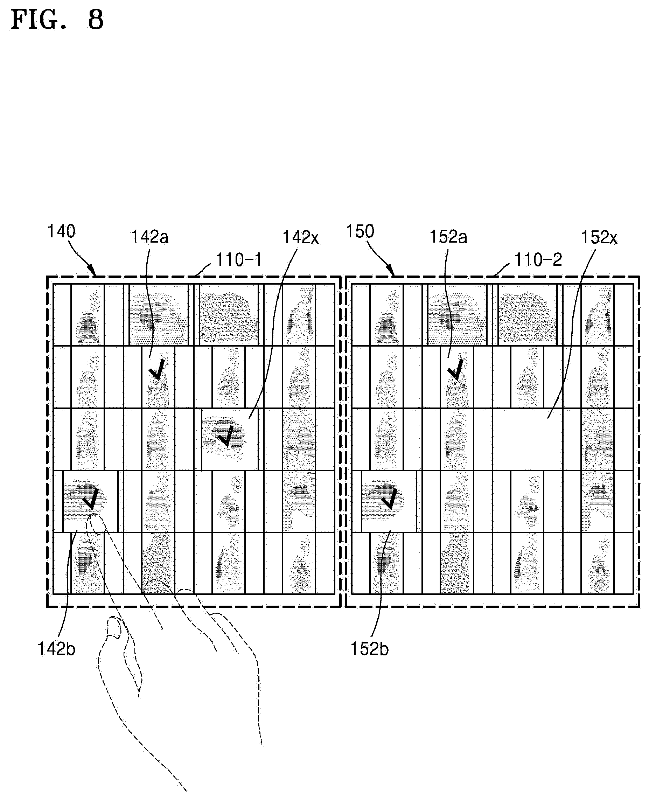

FIG. 8 is a diagram for explaining a method of processing an MR image when a user selects the MR image, according to an exemplary embodiment.

Referring to FIG. 8, the plurality of first MR images 140 are classified into a plurality of series and displayed in the first region 110-1 of a screen, and the plurality of second MR images 150 are classified into the plurality of series and displayed in the second region 110-2 of the screen. According to an exemplary embodiment, if a user selects one or more series, i.e., a plurality of series in the first region 110-1, a plurality of series respectively corresponding to the selected one or more series may be selected in the second region 110-2 without an input. For example, if the user selects series 1-1 142a and 1-2 142b arranged and displayed in the first region 110-1, series 2-1 152a and 2-2 152b respectively corresponding to the series 1-1 142a and 1-2 142b are selected in the second region 110-2.

According to an exemplary embodiment, if the user selects one series in the first region 110-1, a series corresponding to the selected series in the second region 110-2. In this case, a blank image may be selected in the second region 110-2. If the user selects a series 1-3 142x arranged and displayed in the first region 110-1, there is no image corresponding to the series 1-3 142x in the second region 110-2 and thus, a blank image 152x is selected at a position in the second region 110-2 corresponding to the series 1-3 142x.

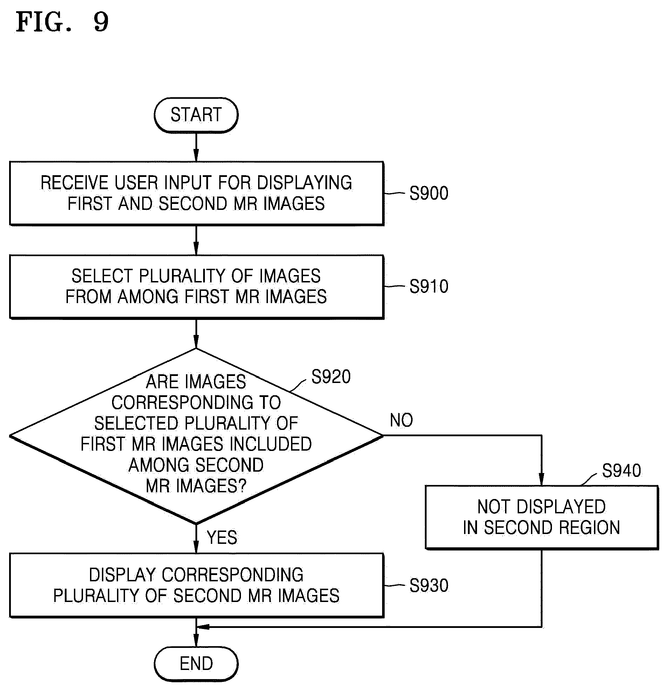

FIG. 9 is a flowchart of a method of processing an MR image when a user selects the MR image, according to an exemplary embodiment.

Referring to FIG. 9, the image processor 200 of view (a) of FIG. 1 receives a user input for displaying a plurality of first MR images and a plurality of second MR images on a screen of the display 100 of view (a) of FIG. 1 (S900). The image processor 200 may include a user input interface. As described with reference to FIG. 2, the first MR images and the second MR images may be MR images acquired by scanning an object of the same patient at different times.

In an exemplary embodiment, in operation S900, the image processor 200 may respectively classify the first MR images and the second MR images into a plurality of series according to a protocol of an object, based on the received user input. Furthermore, the image processor 200 may classify the second MR images into a plurality of series respectively corresponding to the series obtained by classifying the first MR images.

The image processor 200 receives a user input for selecting a plurality of images from among the first MR images (S910). According to an exemplary embodiment, in operation S900, the image processor 200 may respectively classify the first MR images and the second MR images into a plurality of series corresponding to one another, and the user may select one or more series from among the series obtained by classifying the first MR images. For example, referring to FIG. 8 together, the image processor 200 may receive a user input for selecting a series 1-1 142a, a series 1-2 142b, and a series 1-3 142x arranged and displayed in the first region 110-1. According to an exemplary embodiment, the user input for selecting one or more series from among the series for the first MRI images may be sequentially input. In this case, the terms series 1-1 142a, series 1-2 142b, and series 1-3 142x are used to distinguish series selected by the user from one another, and are not limited to representing the order in which a user input is selected.

The image processor 200 determines whether the second MR images include a plurality of images corresponding to the selected plurality of the first MR images (S920). In an exemplary embodiment, the image processor 200 may determine whether there are series in the series for the second MR images corresponding to the one or more series in the series for the first MR images selected in operation S910. For example, referring to FIG. 8 together, if the user selects the series 1-1 142a and the series 1-2 142b, the image processor 200 may determine whether a series corresponding to the series 1-1 141a or series 1-2 141x is included among the series for the second MR images arranged and displayed in the second region 110-2.

If the second MR images include a plurality of images corresponding to the selected plurality of the first MR images, the image processor 200 selects the images corresponding thereto, and the display 100 displays the corresponding selected plurality of second MR images (S930). According to an exemplary embodiment, if there are series in the series for the second MR images corresponding to the one or more series in the series for the first MR images selected in operation S910, the image processor 200 may select the series corresponding thereto, and the display 100 may display the selected series on the screen. Referring to FIG. 8 together, if the image processor 200 receives a user input for selecting the series 1-1 142a and the series 1-2 142b, the image processor 200 may select series 2-1 152a and series 2-2 152b respectively corresponding to the series 1-1 142a and 1-2 142b from among the second MR images arranged and displayed in the second region 110-2. Furthermore, the display 100 may display the series 2-1 151a and 2-2 152b in the second region 110-2.

On the other hand, if the image processor 200 determines that the second MR images does not include a plurality of images corresponding to the selected plurality of the first MR images, the display 100 displays no content at positions in the second region 110-2 respectively corresponding to the selected plurality of the first MR images (S940). According to an exemplary embodiment, if a series corresponding to a series in the series for the first MR images selected by the user is not found among the series for the second MR images, the display 100 may display a blank image in the second region 110-2. Referring to FIG. 8 together, if the image processor 200 receives a user input for selecting the series 1-3142x, the display 100 may display the blank image 152x having no content in a region corresponding to the series 1-3 142x among the series for the second MR images. If one or more series respectively corresponding to one or more series selected via a received user input for selecting one or more series from among the series for the first MR images are not found among the series for the second MR images, the display 100 may display one or more blank images in the second region 110-2.

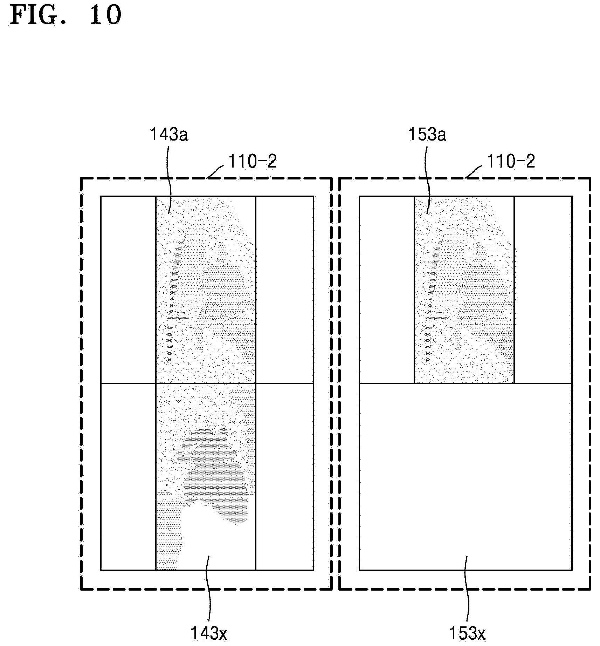

FIG. 10 is a diagram for explaining a method of displaying an MR image according to an exemplary embodiment.

Referring to FIG. 10, two series, i.e., series 1-1 143a and 1-2 143x selected by a user from among a plurality of series obtained by classifying a plurality of first MR images are displayed in a first region 110-1 of the display 100 of view (a) of FIG. 1. Some of a plurality of series obtained by classifying a plurality of second MR images, which respectively correspond to the two series 1-1 143a and 1-2 143x selected by the user among the series for the first MR images, may be displayed in a second region 110-2.

The selected series 1-1 143a and 1-2 143x may be enlarged to completely fill the first region 110-1, and is displayed in the first region 110-1. Furthermore, a series 2-1 153a corresponding to one of the selected 1 series 1-1 143a and 1-2 143x, among the series for the second MR images, may be enlarged to completely half of the second region 110-2 to be displayed.

A blank image 153x is displayed in a portion of the second region 110-2 corresponding to the other half of the second region 110-2 where the series 2-1 153a is not displayed. In detail, because the second images include the series 2-1 153a corresponding to the series 1-1 143a selected by the user from among the images for the first MR images, the series 2-1 153a may be located and displayed in the second region 110-2. However, because there is no series corresponding to the series 1-2 143x in the second region 110-2, only a blank image 153x may be displayed in the remaining portion of the second region 110-2 where the series 2-1 153a is not displayed.

While FIG. 10 shows that two series are enlarged and displayed on two equal-area portions into which each of the first and second regions 110-1 and 110-2 is equally partitioned, exemplary embodiments are not limited thereto. For example, if only one of the series for the first MR images is selected, only the selected one series may be displayed in the whole first region 110-1. Furthermore, if three of the series for the first MR images are selected, the selected three series may respectively be displayed on three equal-area portions into which the first region 110-1 is equally divided.

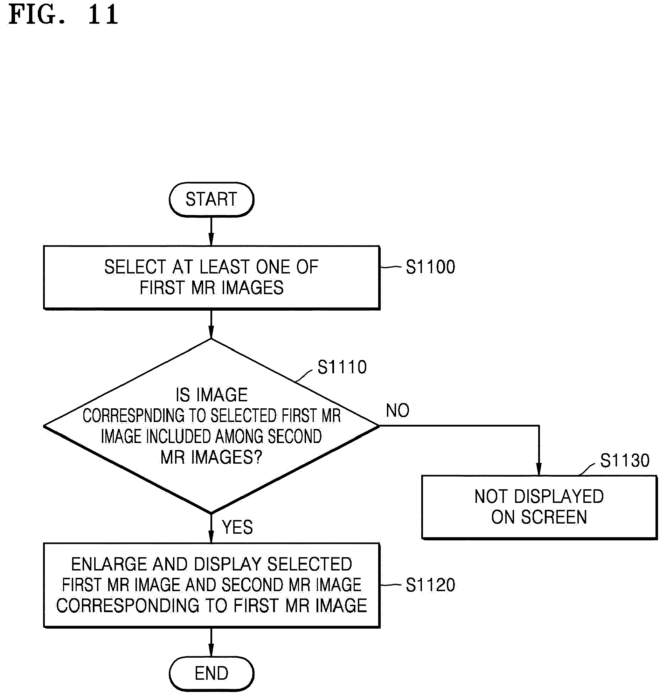

FIG. 11 is a flowchart of a method of displaying an MR image according to an exemplary embodiment.

Referring to FIG. 11, the image processor 200 receives a user input for selecting at least one of a plurality of first MR images (S1100). In an exemplary embodiment, the image processor 200 may classify the first MR images into a plurality of series according to a protocol for an object. The image processor 200 may receive a user input for selecting at least one series from among the plurality series obtained by classifying the first MR images. For example, referring to FIG. 10 together, the image processor 200 may receive a user input for selecting the series 1-1 143a and 1-2 143x from among the series for the first MR images.

The image processor 200 determines whether the second MR images include an image corresponding to the selected first MR image among the first MR images (S1110). According to an exemplary embodiment, the image processor 200 may determine whether a series corresponding to a series in the series for the first MR images selected in operation S1100 is included among the series for the second MR images. For example, referring to FIG. 10 together, if the user selects the series 1-1 143a and 1-2 143x, the image processor 200 may determine whether a series corresponding to the series 1-1 143a or series 1-2 143x is included among the series for the second MR images arranged and displayed in the second region 110-2.

If the second MR images include an image corresponding to the selected first MR image among the first MR images, the image processor 200 selects the image corresponding thereto, and the display 100 enlarges and displays the selected first MR image and the selected second MR image corresponding the selected first MR image (S1120). According to an exemplary embodiment, if a series corresponding to at least one series in the series for the first MR images selected in operation S1100 is included among the series for the second MR images, the image processor 200 may select the series corresponding thereto, and the display 100 may display only the selected series on the screen. The display 100 may enlarge only the at least one series selected from among the series or the first MR images and display an enlarged version of series on the first region 110-1. Referring back to FIG. 10 together, if the image processor 200 receives a user input for selecting the series 1-1 143a, the image processor 200 may select series 2-1 153a corresponding to the series 1-1 143a from among the second MR images arranged and displayed in the second region 110-2. Furthermore, the display 100 may display the series 2-1 153a in the second region 110-2.

On the other hand, if the image processor 200 determines that the second MR images does not include an image corresponding to the selected first MR image among the first MR images, the display 100 displays no content at positions in the second region 110-2 on the screen respectively corresponding to the selected first MR image (S1130). According to an exemplary embodiment, if a series corresponding to at least one series for the first MR images is not found among the series for the second MR images, the display 100 may display a blank image in the second region 110-2. Referring to FIG. 10 together, if the image processor 200 receives a user input for selecting the series 1-2 143x, the display 100 may display the blank image 153x having no content at a position corresponding to the series 1-2 143x from among the series for the second MR images arranged and displayed in the second region 110-2.

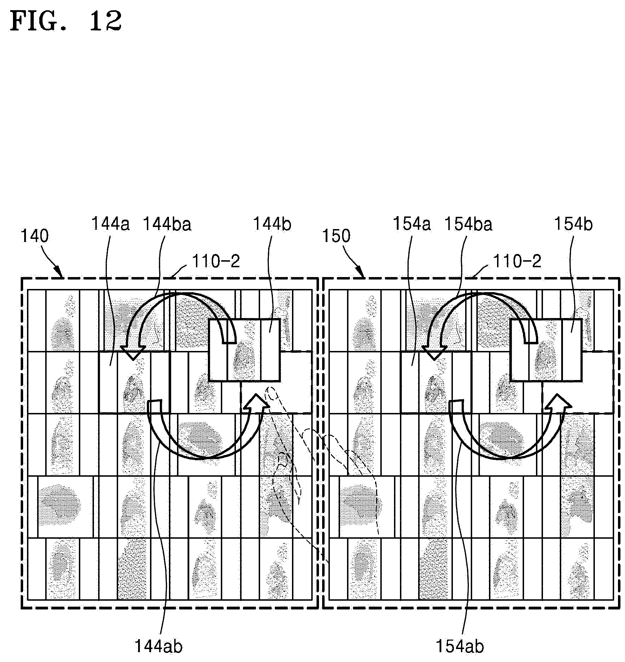

FIG. 12 is a diagram for explaining a method of changing an order in which MR images are arranged, according to an exemplary embodiment.

Referring to FIG. 12, a plurality of series obtained by classifying the plurality of first MR images 140 is displayed in the first region 110-1 of the display 100 of view (a) of FIG. 1, and a plurality of series obtained by classifying the plurality of second MR images 150 is displayed in the second region 110-2 thereof. The MRI apparatus 1000 of view (a) of FIG. 1 or 1B according to the exemplary embodiments may be configured to receive a user input for changing the order in which the series for the first MR images 140 are arranged. For example, if the MRI apparatus 1000 receives an input for selecting series 1-2 144b from among the series for the first MR images 140 displayed in the first region 110-1 and changing a position where the series 1-2 is located to a position where a series 1-1 144a is located, the MRI apparatus 1000 changes the position where the series 1-2 144b is displayed to the position where the series 1-1 144a is displayed (see 144ba). In this case, positions where series 2-1 154a and 2-2 154b in the series for the second MR images 150, respectively corresponding to the series 1-1 144a and 1-2 144b, are displayed are changed accordingly without a separate user input (see 154ba).

When receiving a user input for changing the order in which the series displayed in the first region 110-1 are arranged, the MRI apparatus 1000 may change the order that the series displayed in the first region 110-1 as well as the order in which the series displayed in the second region 110-2 are arranged. It is also possible to change not only the above-described order but also the arbitrary order. For example, if the MRI apparatus 1000 receives an input for selecting the series 1-1 144a from among the series for the first MR images 140 displayed in the first region 110-1 and changing the position where the series 1-1 144a is located to the position where the series 1-2 144b is located, the MRI apparatus 1000 changes the position where the series 1-1 144a is displayed to the position where the series 1-2 144b is displayed (see 144ab). In this case, positions where the series 2-1 154a and 2-2 154b in the series for the second MR images 150, respectively corresponding to the series 1-1 144a and 1-2 144b, are displayed are changed accordingly without a separate user input (see 154ab).

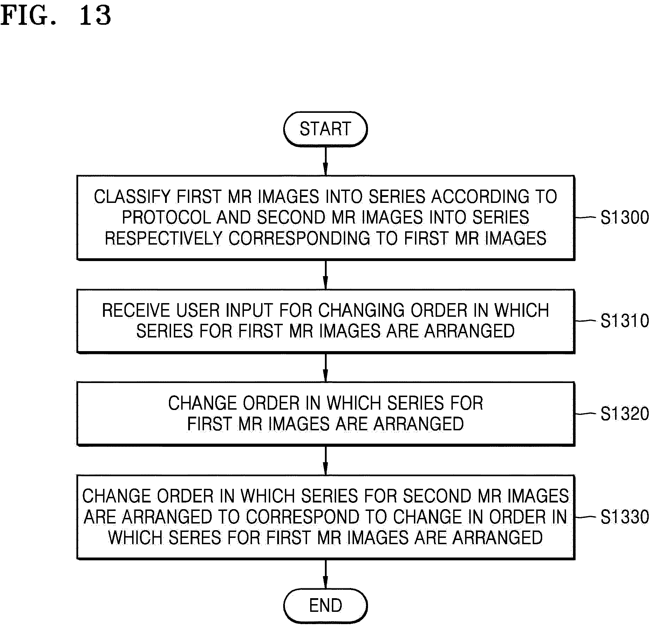

FIG. 13 is a flowchart of a method of changing an order in which MR images are arranged, according to an exemplary embodiment.

Referring to FIG. 13, the image processor 200 of FIG. 2 classifies a plurality of first MR images into a plurality of series according to a protocol of an object and a plurality of second MR images into a plurality of series respectively corresponding to the series for the first MR images (S1300). In an exemplary embodiment, the image processor 200 may arrange the series for the first MR images in the same order as the series for the second MR images.

The image processor 200 receives a user input for changing an order in which the series for the first MR images are arranged (S1310). Referring to FIG. 12 together, in an exemplary embodiment, the image processor 200 may receive a first user input for selecting the series 1-2 144b from among the series for the first MR images and a second user input for changing a position where the series 1-2 is located to a position where the series 1-1 144a is located and vice versa. Furthermore, in another exemplary embodiment, the image processor 200 may receive a third user input for selecting the series 1-1 144a and changing the position where the series 1-1 144a is displayed to the position where the series 1-2 144b is displayed.

The user input received by the image processor 200 may be a screen touch input, an input via an external input device, etc. According to an exemplary embodiment, the display 100 may include a screen consisting of a touch screen and may receive a screen touch input. If a user touches a series whose position is to be changed via an input interface, the display 100 may change the position where the series is located via a GUI. However, the user input received by the image processor 200 is not limited to the above-described examples.

The image processor 200 changes a position where a series selected from among the series for the first MR images via a user input is located, and thus, the order in which the series for the first MR images are arranged (S1320). Referring to FIG. 12 together, the image processor 200 may change positions where the series 1-1 144a and 1-2 144b in the series for the first MR images are located, and thus, an order in which the series for the first MR images are arranged. The display 100 may display the series for the first MR series that are arranged in a changed order.

The image processor 200 changes an order in which the series for the second MR images are arranged to correspond to the change in the order in which the series for the first MR images are arranged (S1330). Referring to FIG. 12 together, if the image processor 200 receives a user input for changing the position where the series 1-2 144b is located to the position where the series 1-1 144a is located, the image processor 200 may change the position in the second region 110-2 where the series 2-2 154b is located to the position where the series 2-1 154a is located.

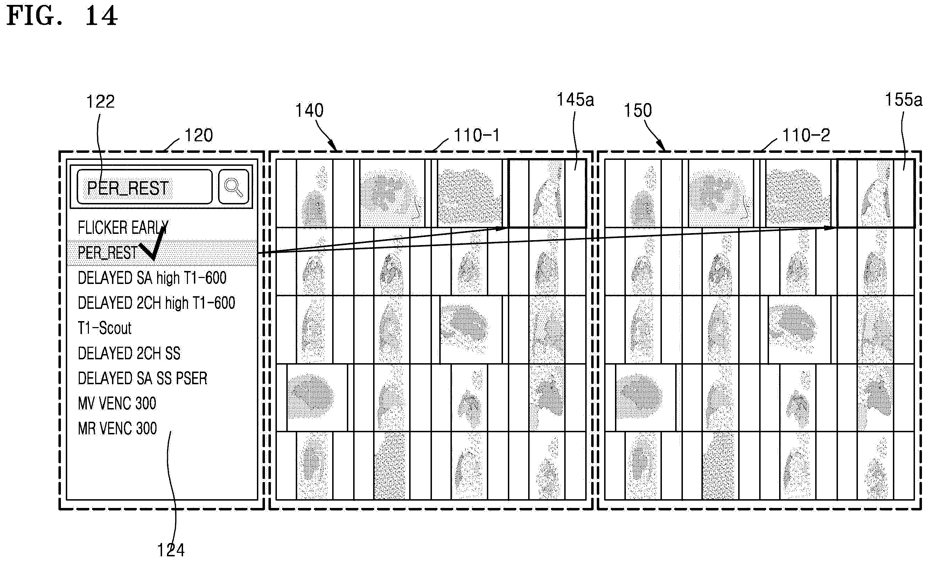

FIG. 14 is a diagram for explaining a method of searching for an MR image according to an exemplary embodiment.

Referring to FIG. 14, a display 100 includes the first region 110-1, the second region 110-2, and a search region 120. The search region 120 includes a search bar 122 and a search result viewer 124. The search bar 122 may receive a user input for selecting a series from among a plurality of series obtained by classifying the plurality of first MR images 140. The search result viewer 124 may display a series found by the user via the search bar 122 among the series for the first MR images. Furthermore, the search bar 122 may receive a user input for selecting a series from among a plurality series obtained by classifying the plurality of second MR images 150, and the search result viewer 124 may display the series that is found among the series for the second MR images 150 based on the received user input. In an exemplary embodiment, the search bar 122 may receive a user input PER_REST for searching for a series related to Rest of Perfusion or PER_REST series, and the search result viewer 124 may display the PER_REST series related to the user input PER_REST in such a manner as to distinguish the PER_REST series from the remaining series not to be searched for.

The image processor 200 of view (a) of FIG. 1 may select a series corresponding to a user's search input received via the search bar 122 from among the series displayed in the first region 110-1. According to an exemplary embodiment, when the search bar 122 receives a user input for searching for PER_SET series, the display 100 may select a first PER_REST series 145a being displayed in the first region 110-1 and display the first PER_REST series 145a to distinguish it from the other series.

The image processor 200 may select a series corresponding to the user's search input received by the search bar 122, among the series displayed in the second region 110-2. For example, the image processor 200 may select series corresponding to a user's search input received by the search bar 122, respectively among the series for the first MR images 140 arranged and displayed in the first region 110-1 and the series for the second MR images 150 arranged and displayed in the second region 110-2, and the display 100 may display the selected series in such a manner as to distinguish them from series not selected therefrom. According to an exemplary embodiment, the image processor 200 may respectively select the first PER_REST series 145a and a second PER_REST series 155a in the first and second regions 110-1 and 110-2, based on the user input PER_REST received by the search bar 122, and the display 100 may display the first PER_REST series 145a and the second PER_REST series 155a.

The MRI apparatus 1000 according to the exemplary embodiments is configured to receive a user input for searching for at least one among the first and second MR images 140 and 150 via the image processor 200 and display the at least one MR image to be searched for in the search region 120. Thus, the time, cost, and effort for the user to manually search for MR images of interest among all the first and second MR images 140 and 150 of an object may be reduced.

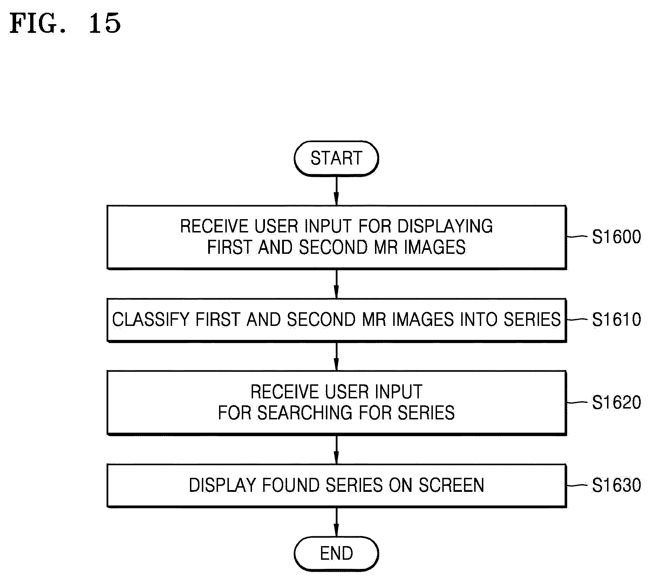

FIG. 15 is a flowchart of a method of searching for an MR image according to an exemplary embodiment.

Referring to FIG. 15, the image processor 200 of view (a) of FIG. 1 receives a user input for displaying a plurality of first MR images and a plurality of second MR images on the display 100 of FIG. 14 (S1600). The image processor 200 may include a user input interface.

The image processor 200 classifies the first MR images into a plurality of series according to a protocol of an object. The image processor 200 also classifies the second MR images into a plurality of series respectively corresponding to the series obtained by classifying the first MR images (S1610).

The image processor 200 receives a user input for searching for a series among the series obtained by classifying the first MR images and the series obtained by classifying the second MR images (S1620).

The image processor 200 selects a series found based on the received user input, and the display 100 displays the selected series on a screen in such a manner as to distinguish it from the other non-selected images (S1630). Referring to FIG. 14 together, the image processor 200 may respectively select the first PER_REST series 145a and the second PER_REST series 155a in the first and second regions 110-1 and 110-2 based on the user input PER_REST for searching for a PER_REST series, which is received by the search bar 122. The display 100 may respectively display the first PER_REST series 145a and the second PER_REST series 155a in the first and second regions 110-1 and 110-2.

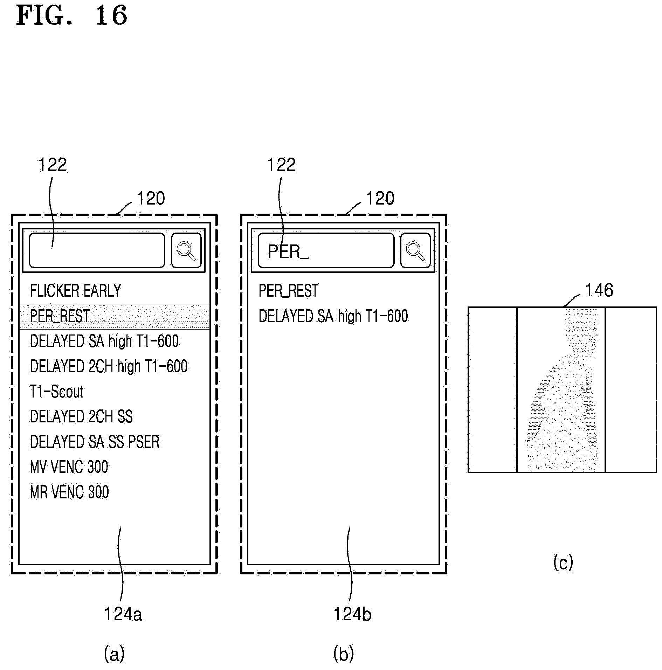

FIG. 16 is a diagram illustrating the search region 120 in an apparatus for processing an MR image, and an enlarged version of the MR image, according to an exemplary embodiment.

Referring to view (a) of FIG. 16, the search region 120 includes the search bar 122 and a search result viewer 124a. The search bar 122 is in a state of not receiving a user input for selecting a series from among a plurality of series respectively obtained by classifying a plurality of first MR images and a plurality of series obtained by classifying a plurality of second MR images. In this case, the search result viewer 124a may display names of all of the series for the first and second MR images.

Referring to view (b) of FIG. 16, the image processor 200 of view (a) of FIG. 1 may receive a user input for selecting a series related to a rest perfusion image. In this case, the user input, i.e., entry of characters "PER_" may be displayed in the search bar 122. Only results related to PER_REST series may be displayed on a search result viewer 124b while series not related to the rest perfusion image may not be displayed thereon.

Referring to view (c) of FIG. 16, the display 100 of FIG. 14 may enlarge only a series found based on a user input received via the image processor 200 of view (a) of FIG. 1 and display an enlarged version of series. According to an exemplary embodiment, the display 100 may enlarge a PER-REST series 146 related to a rest perfusion image to be displayed.

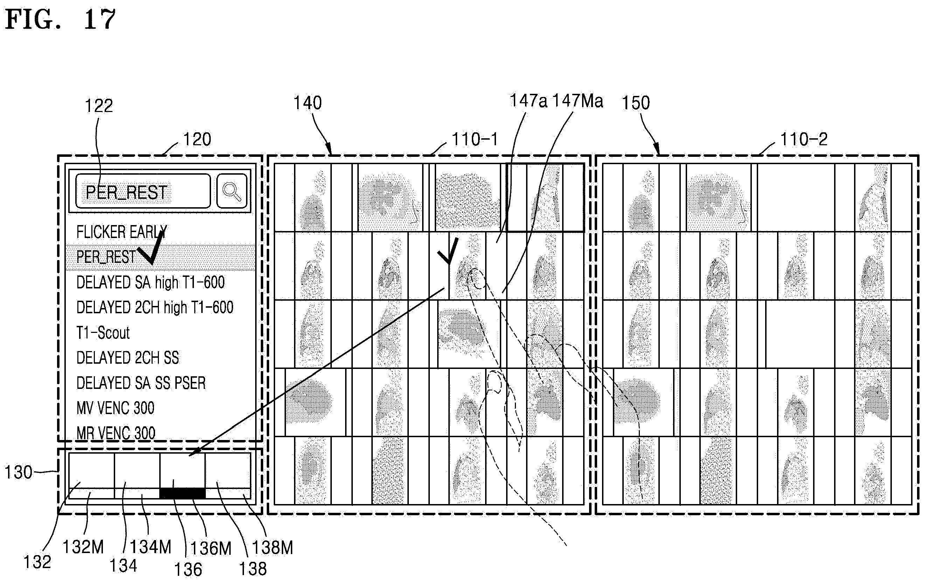

FIG. 17 is a diagram for explaining a method of executing an application using an MR image according to an exemplary embodiment.

Referring to FIG. 17, the display 100 includes the first region 110-1, the second region 110-2, the search region 120, and an application display region 130. A plurality of icons for respectively executing a plurality of applications may be displayed in the application display region 130. On the display 100, a first identification mark 147Ma used to distinguish the series 1-1 147a from the other non-selected series may be indicated on a series 1-1 147a selected via a user input from among a plurality of series into which a plurality of first MR images 140 are classified. In the application display region 130, an identification mark 136M identical to the first identification mark 147Ma may be indicated on a third application.

First through fourth application icons 132, 134, 136, and 138 are displayed in the application display region 130. However, the number of application icons displayed in the application display region 130 is not limited to 4. Application identification marks may respectively be displayed at lower ends of the first through fourth application icons 132, 134, 136, and 138. The application identification marks includes first through fourth application identification marks 132M, 134M, 136M, and 138M. If a series is selected from among a plurality of series obtained by classifying the first MR images 140 and a plurality of series obtained by classifying the second MR images 150, an identification mark corresponding to an application icon for performing quantitative data analysis on an object by using the selected series may be displayed. According to an exemplary embodiment, if the user selects the series 1-1 147a, the third application identification mark 136M corresponding to the third application icon 136 executing an application capable of performing quantitative data analysis on the object by using the selected series 1-1 147a may be displayed using the same indication as the first identification mark 147Ma. The first identification mark 147Ma may be indicated in the same color, design, or pattern as the third application identification mark 136M so that the selected series is distinguishable from the other non-selected series or non-selected applications.

The MRI apparatus 1000 according to the exemplary embodiments is configured to display an application capable of performing quantitative analysis of the object based on an MR image selected by the user and indicate the same identification mark as for a series for the selected MR image on the application, thereby allowing the user to easily select or execute the application.

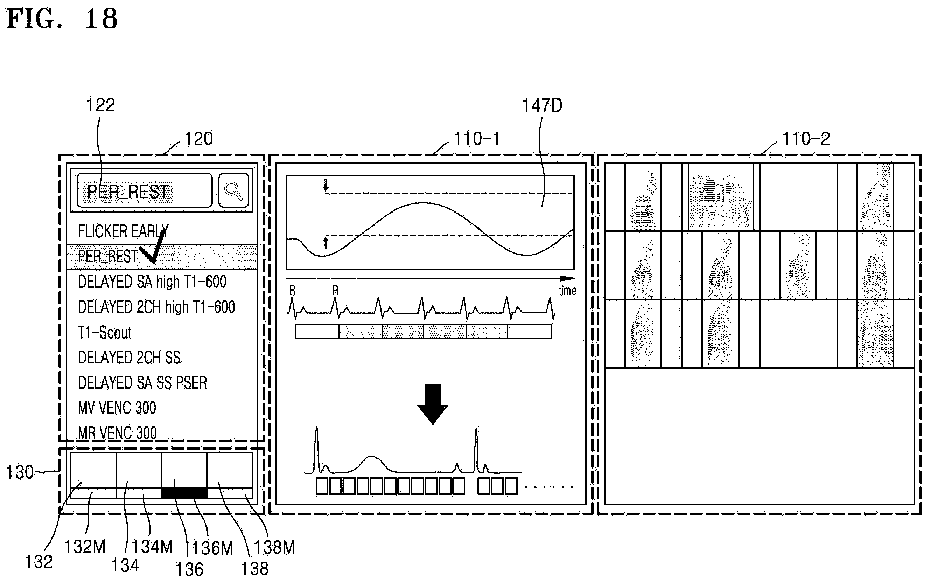

FIG. 18 is a diagram for explaining a method of displaying analysis data for an object obtained by executing an MR image processing application, according to an exemplary embodiment.

Referring to FIG. 18, the display 100 includes the first region 110-1, the second region 110-2, the search region 120, and the application display region 130. Quantitative analysis data 147D for the object obtained by executing an application may be displayed in the first region 110-1. According to an exemplary embodiment, the quantitative analysis data 147D may be enlarged to completely fill the first region 110-1 and displayed in the first region 110-1.

According to an exemplary embodiment, the image processor 200 of view (a) of FIG. 1 may receive a user input for selecting a series related to a rest perfusion image and the third application icon 136. Furthermore, the display 100 may display first analysis data of the object obtained via a third application in the first region 110-1.

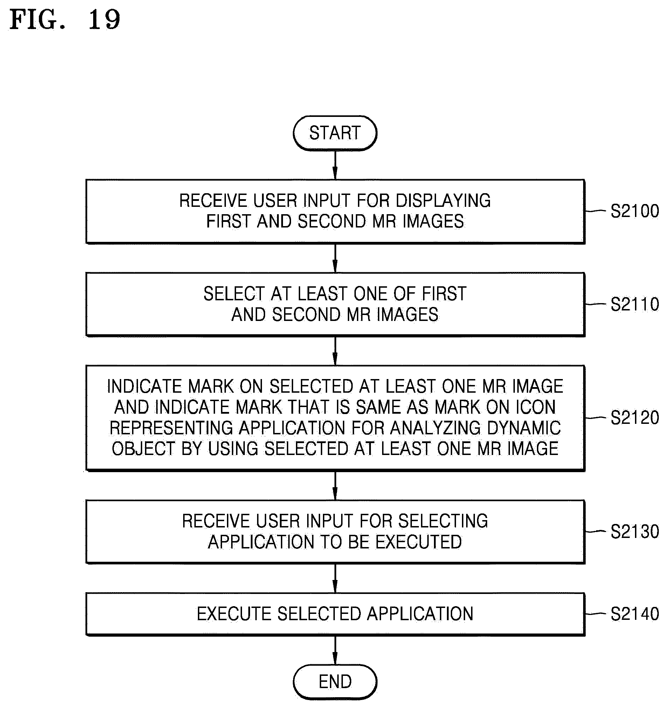

FIG. 19 is a flowchart of a method of executing an MR image processing application according to an exemplary embodiment.

The image processor 200 of view (a) of FIG. 1 receives a user input for displaying a plurality of first MR images and a plurality of second MR images on the display 100 of view (a) of FIG. 1 (S2100). The image processor 200 may include a user input interface.

According to an exemplary embodiment, in operation S2100, the image processor 200 may respectively classify the first and second MR images into a plurality of series according to a protocol of an object based on the received user input. Alternatively, the image processor 200 may classify the second MR images into a plurality of series respectively corresponding to a plurality of series obtained by classifying the first MR images.

The image processor 200 receives a user input for selecting at least one series from among the series obtained by classifying the first and second MR images, respectively (S2110). Referring to FIG. 17 together, according to an exemplary embodiment, the image processor 200 may receive a user input for selecting the series 1-1 147a.

The display 100 may indicate an identification mark on the selected at least one series and also indicate a mark that is the same as the identification mark on an icon representing an application for analyzing the dynamic object by using the selected at least one series (S2120). Referring to FIG. 18 together, according to an exemplary embodiment, the display 100 may indicate the first identification mark 147Ma on the series 1-1 147a selected by the user and display the third application identification mark 136M, which identifies the third application for performing quantitative analysis of the object by using the selected series 1-1, in the same manner as the first identification mark 147Ma. The first identification mark 147Ma may be displayed in the same color as the third application identification mark 136M.

The image processor 200 receives a user input for selecting an application to be executed (S2130). Referring to FIG. 17 together, the image processor 200 may receive a user input for selecting and inputting the third application icon 136 to execute the third application. For example, the user input may be a screen touch input, an input via an external input device, etc.

The image processor 200 executes the application selected via the user input (S2140). Referring to FIG. 17 together, the image processor 200 may execute the third application according to the user input for selecting the third application icon 136. The display 100 may display the quantitative analysis data 147D obtained by executing the third application in the first region 110-1.

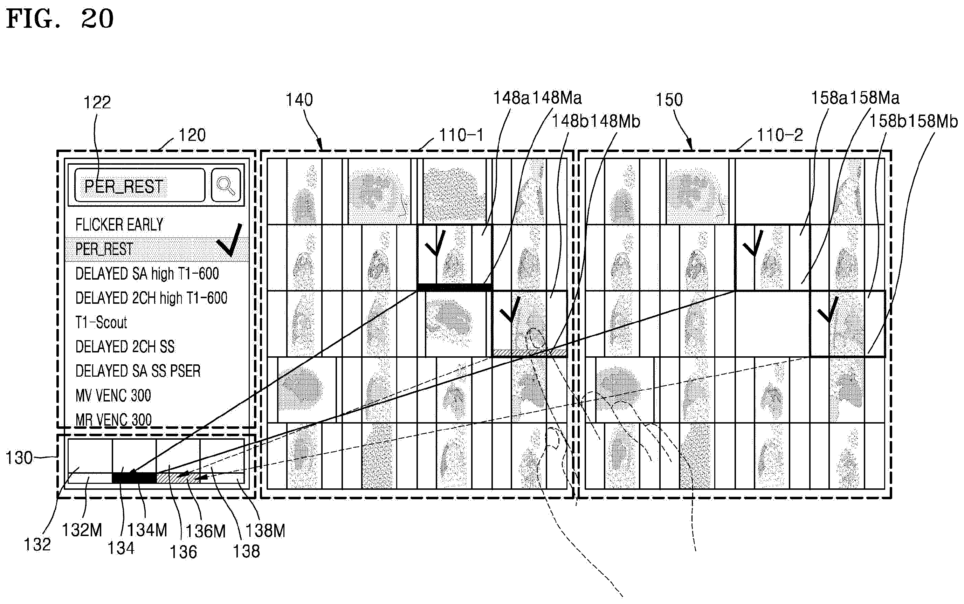

FIG. 20 is a diagram for explaining a method of executing an application using an MR image according to an exemplary embodiment.

Referring to FIG. 20, the display 100 includes the first region 110-1, the second region 110-2, the search region 120, and the application display region 130. A plurality of series into which the plurality of first MR images 140 are classified are displayed in the first region 110-1. A plurality of series into which the plurality of second MR images 150 are classified are displayed in the second region 110-2. The search bar 122 is displayed in the search region 120. The first through fourth icons 132, 134, 136, and 138 for respectively executing a plurality of applications are displayed in the application display region 130. In the first region 110-1, a series 1-1 148a and a series 1-2 148b may be selected from among the series obtained by classifying the first MR images 140 via a user input. In the second region 110-2, a series 2-1 158a and a series 2-2 158b may also be selected from among the series obtained by classifying the second MR images 150 via the user input.

The series 1-1 148a and series 1-2 148b selected via the user input from among the series for the first MR images 140 may be displayed in the first region 110-1, together with identification marks 1-1 148Ma and 1-2 148Mb respectively identifying the series 1-1 148a and series 1-2 148b. The series 2-1 158a and series 2-2 158b respectively corresponding to the selected series 1-1 148a and 1-2 148b, among the series for the second MR images 150, may be displayed in the second region 110-2. Furthermore, identification marks 2-1 158Ma and 2-2 158Mb respectively identifying the series 2-1 158a and 2-2 158b may be displayed in the second region 110-2.

The first through fourth application icons 132, 134, 136, and 138 and the first through application identification marks 132M, 134M, 136M, and 138M are displayed in the application display region 130. According to an exemplary embodiment, if at least one series is selected from among the series obtained by classifying the first MR images 140 and the series obtained by classifying the second MR image 150, an application identification mark corresponding to an application icon for performing quantitative data analysis on an object by using the selected at least one series may be displayed. For example, if an application capable of performing quantitative data analysis of the object by using the series 1-1 148a is a second application, the second application identification mark 134M may be displayed in the same manner as the identification mark 1-1 148Ma for the series 1-1 148a. In an exemplary embodiment, because a series corresponding to the series 1-1 148a among the series for the second MR images 150 is the series 2-1 158a, the identification mark 2-1 158Ma may be displayed in the same manner as the second application identification mark 134M. Furthermore, if an application capable of executing quantitative data analysis of the object by using the series 1-2 148b is a third application, the third application identification mark 136M may be displayed in the same manner as the identification mark 1-2 148Mb for the series 1-2 148b. Similarly, because a series corresponding to the series 1-2 148b among the series for the second MR images 150 is the series 2-2 158b, the identification mark 2-2 158Mb may be displayed in the same manner as the third application identification mark 136M.

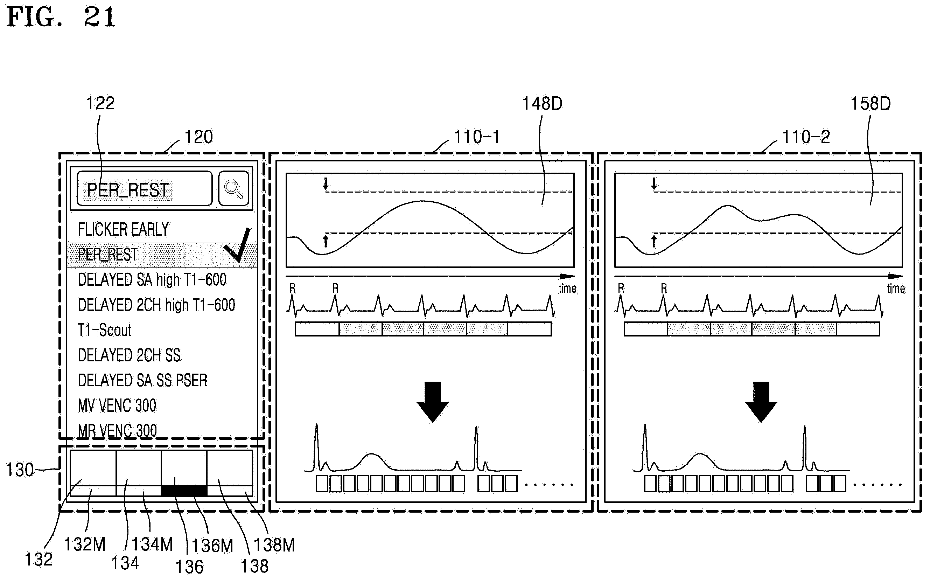

FIG. 21 is a diagram for explaining a method of displaying analysis data for an object obtained by executing an MR image processing application, according to an exemplary embodiment.

Referring to FIG. 21, the display 100 includes the first region 110-1, the second region 110-2, the search region 120, and the application display region 130. Pieces of quantitative analysis data for the object obtained by executing an application may respectively be displayed in the first and second regions 110-1 and 110-2. According to an exemplary embodiment, the pieces of quantitative analysis data may be enlarged to completely fill the first and second regions 110-1 and 110-2, respectively, and displayed therein.

According to an exemplary embodiment, the image processor 200 of view (a) of FIG. 1 may receive a user input for selecting a series related to a rest perfusion image from among a plurality of series obtained by classifying a plurality of first MR images 140 and a plurality of series obtained by classifying a plurality of second images 150. Furthermore, the image processor 200 may receive a user input for selecting a third application icon 136. Referring to FIG. 20 together, the image processor 200 may receive a user input for selecting the series 1-2 148b and select the series 2-2 158b corresponding to the series 1-2 148b. The display 100 respectively displays first analysis data 148D and second analysis data 158D for the object obtained by executing a third application using the series 1-2 148b and 2-2 158b in the first and second regions 110-1 and 110-2. Due to this configuration, the user is able to analyze the first analysis data 148D and the second analysis data 158D within a single screen and thus, easily detect progress of a change in a state of the object or a difference between lesions, etc.

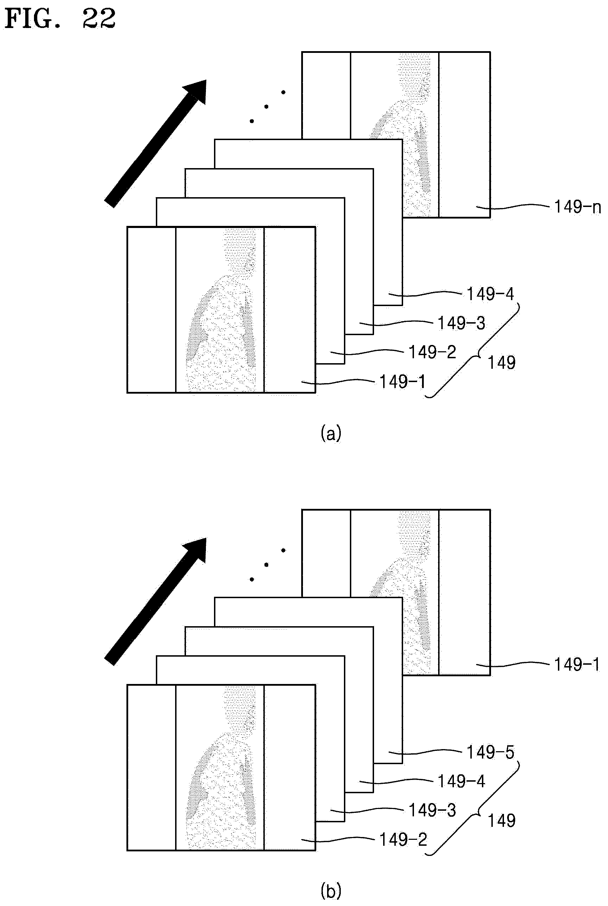

FIG. 22 is a diagram for explaining a method of sequentially displaying MR images according to an exemplary embodiment.

Referring to view (a) of FIG. 22, a series 1-1 149 that is one from among a plurality of series into which a plurality of first MR images are classified may include a plurality of MR images. The series 1-1 149 may include n MR images, i.e., first through n-th MR images 149-1 through 149-n. In an exemplary embodiment, n is an integer greater than or equal to 2.

The MR images in the series 1-1 149 may be arranged in an order from the first MR image 149-1 to the n-th MR image 149-n.

Referring to view (b) of FIG. 22, the first MR image 149-1 may be moved next to the n-th MR image 149-n, and the second MR image 149-2 may be moved to an original position where the first MR image 149-1 has been located. Thereafter, the second MR image may be moved next to the first MR image 149-1, and the third MR image 149-3 may be moved to an original position where the second MR image 149-2 has been located.

According to an exemplary embodiment, the MR images in the series 1-1 149 may be displayed sequentially over time. In an exemplary embodiment, the MR images 149-1 through 149-n in the series 1-1 149 may be displayed sequentially on a screen in a similar way to when a moving image is played back.

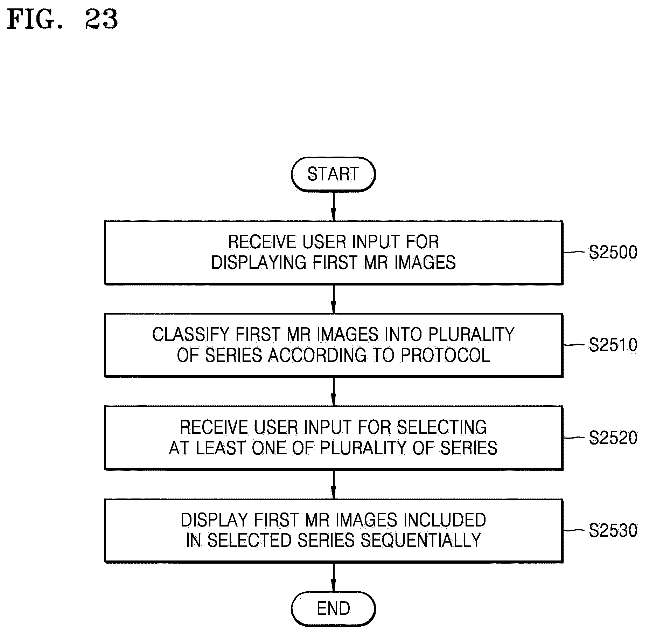

FIG. 23 is a flowchart of a method of sequentially displaying MR images according to an exemplary embodiment.

Referring to FIG. 23, the image processor 200 of FIG. 1 receives a user input for displaying a plurality of first MR images on the display 100 of view (a) of FIG. 1 (S2500). The image processor 200 may include a user input interface.

The image processor 200 classifies the first MR images into a plurality of series according to a protocol of an object (S2510).

The image processor 200 receives a user input for selecting at least one of the series obtained by classifying the first MR images (S2520). Referring to the views (a) and (b) of FIG. 22 together, the selected at least one series may be series 1-1 149.

The display 100 sequentially displays a plurality of MR images included in the series selected in operation S2520 over time (S2530). Referring to the views (a) and (b) of FIG. 22 together, the MR images in the series 1-1 149 may be displayed sequentially over time. According to an exemplary embodiment, the MR images 149-1 through 149-n in the series 1-1 149 may be displayed sequentially on the screen like a moving image being played back.

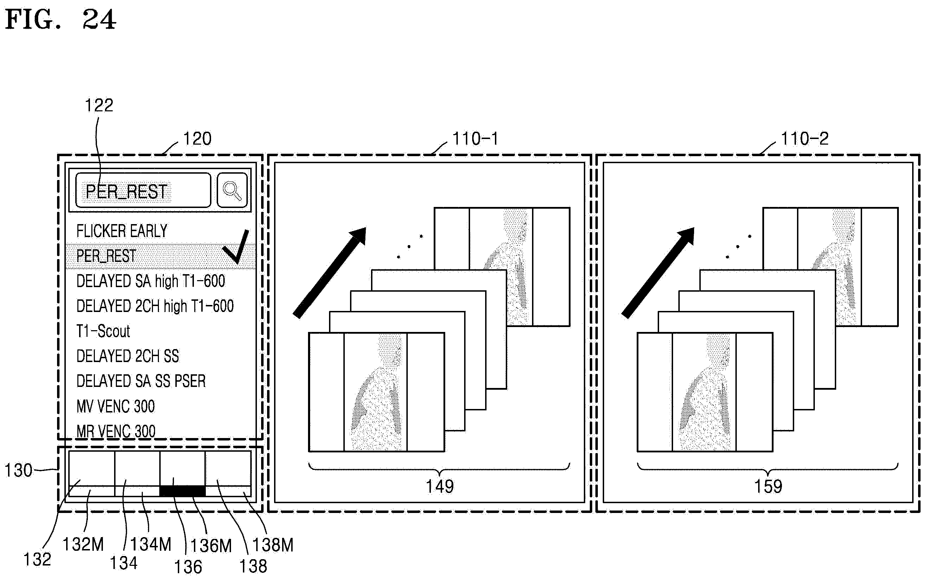

FIG. 24 is a diagram for explaining a method of sequentially displaying MR images according to an exemplary embodiment.

Referring to FIG. 24 together with the views (a) and (b) of FIG. 22, the MR images 149-1 through 149-n in the 1-1-series 149 may be displayed in the first region 110-1 of the screen of the display 100. A series 2-1 159 corresponding to the series 1-1 149 among a plurality of series obtained by classifying a plurality of second MR images may include a plurality of MR images 159-1 through 159-n that may be displayed in a second region 110-2 of the screen.

The MR images 149-1 through 149-n in the series 1-1 149 may be displayed sequentially in the first region 110-1 over time, like a moving image being played back. Similarly, the MR images 159-1 through 159-n in the series 2-1 159 may be displayed sequentially in the second region 110-2 over time, like a moving image being played back.

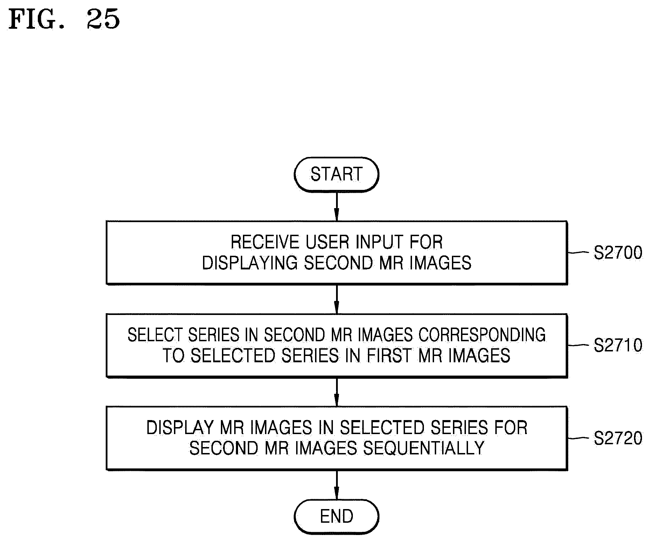

FIG. 25 is a flowchart of a method of sequentially displaying MR images according to an exemplary embodiment.

FIG. 25 may be a flowchart of the method that is applied after operation of sequentially displaying a plurality of MR images (e.g., 149-1 through 149-n) in a selected series over time as described above with reference to FIG. 23.

Referring to FIG. 25, the image processor 200 of view (a) of FIG. 1 receives a user input for displaying a plurality of second MR images on the display 100 of view (a) of FIG. 1 (S2700). The image processor 200 may include a user input interface.

The image processor 200 selects a series in a plurality of series obtained by classifying the second MR images corresponding to the series selected in the series for the first MR images in operation S2520 of FIG. 23 (S2710). Referring to FIG. 24 together, according to an exemplary embodiment, the image processor 200 may select the series 2-1 159 corresponding to the series 1-1 149 from among the series for the second MR images.

The display 100 sequentially displays a plurality of MR images in the series selected from among the series for the second MR images in operation S2710 over time (S2720). Referring to FIG 24 together, the display 100 may display the MR images 159-1 through 159-n in the series 2-1 159 in the second region 110-2 sequentially with respect to time.

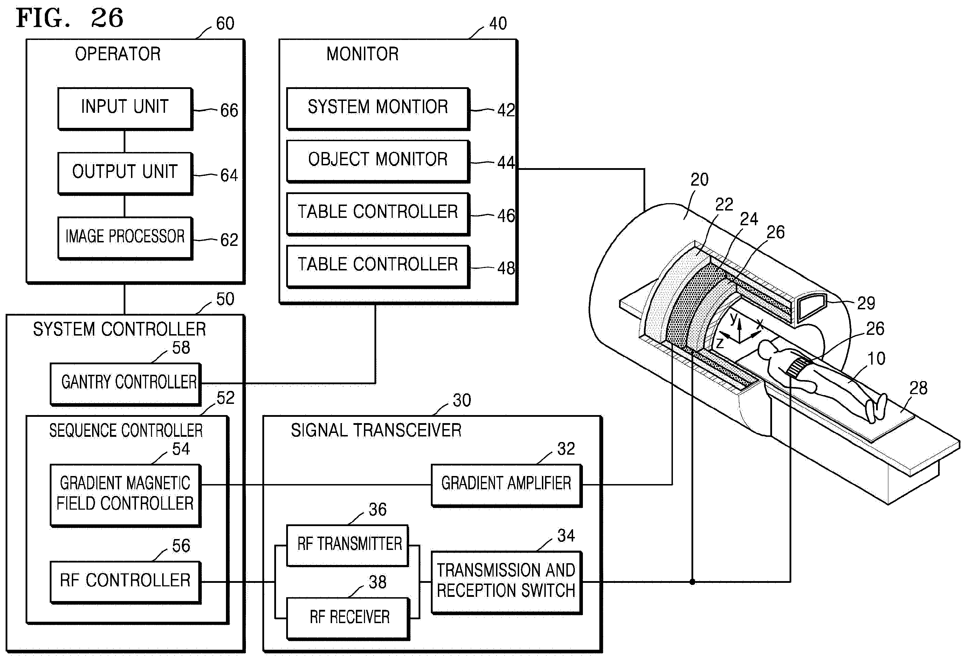

FIG. 26 is a block diagram of an MRI system. Referring to FIG. 26, the MRI system includes a gantry 20, a signal transceiver 30, a monitor 40, a system controller 50, and an operator 60.

The gantry 20 prevents external emission of electromagnetic waves generated by a main magnet 22, a gradient coil 24, and an RF coil 26. A magnetostatic field and a gradient magnetic field are formed in a bore in the gantry 20, and an RF signal is emitted toward an object 10.

The main magnet 22, the gradient coil 24, and the RF coil 26 may be arranged in a predetermined direction of the gantry 20. The predetermined direction may be a coaxial cylinder direction. The object 10 may be disposed on a table 28 that is capable of being inserted into a cylinder along a horizontal axis of the cylinder.

The main magnet 22 generates a magnetostatic field or a static magnetic field for aligning magnetic dipole moments of atomic nuclei of the object 10 in a constant direction. A precise and accurate MR image of the object 10 may be obtained due to a magnetic field generated by the main magnet 22 being strong and uniform.

The gradient coil 24 includes X, Y, and Z coils for generating gradient magnetic fields in X-, Y-, and Z-axis directions crossing each other at right angles. The gradient coil 24 may provide location information of each region of the object 10 by differently inducing resonance frequencies according to the regions of the object 10.

The RF coil 26 may emit an RF signal toward a patient and receive an MR signal emitted from the patient. In detail, the RF coil 26 may transmit, toward atomic nuclei included in the patient and having precessional motion, an RF signal having the same frequency as that of the precessional motion, stop transmitting the RF signal, and then receive an MR signal emitted from the atomic nuclei included in the patient.

For example, to transit an atomic nucleus from a low energy state to a high energy state, the RF coil 26 may generate and apply an electromagnetic wave signal that is an RF signal corresponding to a type of the atomic nucleus, to the object 10. When the electromagnetic wave signal generated by the RF coil 26 is applied to the atomic nucleus, the atomic nucleus may transit from the low energy state to the high energy state. Then, when electromagnetic waves generated by the RF coil 26 disappear, the atomic nucleus to which the electromagnetic waves were applied transits from the high energy state to the low energy state, thereby emitting electromagnetic waves having a Lamor frequency. In other words, when the applying of the electromagnetic wave signal to the atomic nucleus is stopped, an energy level of the atomic nucleus is changed from a high energy level to a low energy level, and thus the atomic nucleus may emit electromagnetic waves having a Lamor frequency. The RF coil 26 may receive electromagnetic wave signals from atomic nuclei included in the object 10.

The RF coil 26 may be realized as one RF transmitting and receiving coil having both a function of generating electromagnetic waves each having an RF that corresponds to a type of an atomic nucleus and a function of receiving electromagnetic waves emitted from an atomic nucleus. Alternatively, the RF coil 26 may be realized as a transmission RF coil having a function of generating electromagnetic waves each having an RF that corresponds to a type of an atomic nucleus, and a reception RF coil having a function of receiving electromagnetic waves emitted from an atomic nucleus.

The RF coil 26 may be fixed to the gantry 20 or may be detachable. When the RF coil 26 is detachable, the RF coil 26 may be an RF coil for a part of the object, such as a head RF coil, a chest RF coil, a leg RF coil, a neck RF coil, a shoulder RF coil, a wrist RF coil, or an ankle RF coil.

The RF coil 26 may communicate with an external apparatus via wires and/or wirelessly, and may also perform dual tune communication according to a communication frequency band.

The RF coil 26 may be a birdcage coil, a surface coil, or a transverse electromagnetic (TEM) coil according to structures.

The RF coil 26 may be a transmission exclusive coil, a reception exclusive coil, or a transmission and reception coil according to methods of transmitting and receiving an RF signal.

The RF coil 26 may be an RF coil having various numbers of channels, such as 16 channels, 32 channels, 72 channels, and 144 channels.

The gantry 20 further includes a display 29 disposed outside the gantry 20 and a display disposed inside the gantry 20. The gantry 20 may provide predetermined information to the user or the object 10 through the display 29 and the display respectively disposed outside and inside the gantry 20.

The signal transceiver 30 may control the gradient magnetic field formed inside the gantry 20, i.e., in the bore, according to a predetermined MR sequence, and control transmission and reception of an RF signal and an MR signal.

The signal transceiver 30 includes a gradient amplifier 32, a transmission and reception switch 34, an RF transmitter 36, and an RF receiver 38.

The gradient amplifier 32 drives the gradient coil 24 included in the gantry 20, and may supply a pulse signal for generating a gradient magnetic field to the gradient coil 24 under the control of a gradient magnetic field controller 54. By controlling the pulse signal supplied from the gradient amplifier 32 to the gradient coil 24, gradient magnetic fields in X-, Y-, and Z-axis directions may be synthesized.

The RF transmitter 36 and the RF receiver 38 may drive the RF coil 26. The RF transmitter 36 may supply an RF pulse in a Lamor frequency to the RF coil 26, and the RF receiver 38 may receive an MR signal received by the RF coil 26.

The transmission and reception switch 34 may adjust transmitting and receiving directions of the RF signal and the MR signal. For example, the transmission and reception switch 34 may emit the RF signal toward the object 10 through the RF coil 26 during a transmission mode, and receive the MR signal from the object 10 through the RF coil 26 during a reception mode. The transmission and reception switch 34 may be controlled by a control signal output by an RF controller 56.

The monitor 40 may monitor or control the gantry 20 or devices mounted on the gantry 20. The monitor 40 includes a system monitor 42, an object monitor 44, a table controller 46, and a display controller 48.

The system monitor 42 may monitor and control a state of the magnetostatic field, a state of the gradient magnetic field, a state of the RF signal, a state of the RF coil 26, a state of the table 28, a state of a device measuring body information of the object 10, a power supply state, a state of a thermal exchanger, and a state of a compressor.

The object monitor 44 monitors a state of the object 10. In detail, the object monitor 44 may include a camera for observing a movement or position of the object 10, a respiration measurer for measuring the respiration of the object 10, an electrocardiogram (ECG) measurer for measuring the electrical activity of the object 10, or a temperature measurer for measuring a temperature of the object 10.

The table controller 46 controls a movement of the table 28 where the object 10 is positioned. The table controller 46 may control the movement of the table 28 according to a sequence control of a sequence controller 50. For example, during moving imaging of the object 10, the table controller 46 may continuously or discontinuously move the table 28 according to the sequence control of the sequence controller 50, and thus the object 10 may be photographed in a field of view (FOV) larger than that of the gantry 20.

The display controller 48 controls the display 29 disposed outside the gantry 20 and the display disposed inside the gantry 20. In detail, the display controller 48 may control the display 29 and the display to be on or off, and may control a screen image to be output on the display 29 and the display. Also, when a speaker is located inside or outside the gantry 20, the display controller 48 may control the speaker to be on or off, or may control sound to be output via the speaker.

The system controller 50 includes the sequence controller 52 for controlling a sequence of signals formed in the gantry 20, and a gantry controller 58 for controlling the gantry 20 and the devices mounted on the gantry 20.

The sequence controller 52 includes the gradient magnetic field controller 54 for controlling the gradient amplifier 32, and the RF controller 56 for controlling the RF transmitter 36, the RF receiver 38, and the transmission and reception switch 34. The sequence controller 52 may control the gradient amplifier 32, the RF transmitter 36, the RF receiver 38, and the transmission and reception switch 34 according to a pulse sequence received from the operator 60. Here, the pulse sequence includes all information used to control the gradient amplifier 32, the RF transmitter 36, the RF receiver 38, and the transmission and reception switch 34. For example, the pulse sequence may include information about a strength, an application time, and application timing of a pulse signal applied to the gradient coil 24.

The operator 60 may request the system controller 50 to transmit pulse sequence information while controlling an overall operation of the MRI system.

The operator 60 includes an image processor 62 for receiving and processing the MR signal received by the RF receiver 38, an output interface 64, and an input interface 66.

The image processor 62 may include the image processors 200 shown in FIG. 1.

The image processor 62 may process the MR signal received from the RF receiver 38 to generate MR image data of the object 10.

The image processor 62 receives the MR signal received by the RF receiver 38 and performs any one of various signal processes, such as amplification, frequency transformation, phase detection, low frequency amplification, and filtering, on the received MR signal.

The image processor 62 may arrange digital data in a k space (for example, also referred to as a Fourier space or a frequency space) of a memory, and rearrange the digital data into image data via 2D or 3D Fourier transformation.

The image processor 62 may perform a composition process or difference calculation process on the image data. The composition process may include an addition process on a pixel or a maximum intensity projection (MIP) process. The image processor 62 may store not only the rearranged image data but also image data on which a composition process or a difference calculation process is performed, in a memory or an external server.

The image processor 62 may perform any of the signal processes on the MR signal in parallel. For example, the image processor 62 may perform a signal process on a plurality of MR signals received by a multi-channel RF coil in parallel to rearrange the MR signals into image data.

The output interface 64 may include the display 100 of view (a) of FIG. 1.

The output interface 64 may output image data generated or rearranged by the image processor 62 to the user. The output interface 64 may also output information for the user to manipulate the MRI system, such as a user interface (UI), user information, or object information. The output interface 64 may be a speaker, a printer, a cathode-ray tube (CRT) display, a liquid crystal display (LCD), a plasma display panel (PDP), an organic light-emitting device (OLED) display, a field emission display (FED), a light-emitting diode (LED) display, a vacuum fluorescent display (VFD), a digital light processing (DLP) display, a flat panel display (FPD), a 3-dimensional (3D) display, a transparent display, or any one of other various output devices that are well known to one of ordinary skill in the art.

The user may input object information, parameter information, a scan condition, a pulse sequence, or information about image composition or difference calculation by using the input interface 66. The input interface 66 may be a keyboard, a mouse, a track ball, a voice recognizer, a gesture recognizer, a touch screen, or any one of other various input devices that are well known to one of ordinary skill in the art.

The signal transceiver 30, the monitor 40, the system controller 50, and the operator 60 are separate components in FIG. 26, but it will be obvious to one of ordinary skill in the art that respective functions of the signal transceiver 30, the monitor 40, the system controller 50, and the operator 60 may be performed by another component. For example, the image processor 62 converts the MR signal received from the RF receiver 38 into a digital signal in FIG. 26, but alternatively, the conversion of the MR signal into the digital signal may be performed by the RF receiver 38 or the RF coil 26.

The gantry 20, the RF coil 26, the signal transceiver 30, the monitor 40, the system controller 50, and the operator 60 may be connected to each other by wire or wirelessly, and when they are connected wirelessly, the MRI system may further include an apparatus for synchronizing clock signals therebetween. Communication between the gantry 20, the RF coil 26, the signal transceiver 30, the monitor 40, the system controller 50, and the operator 60 may be performed by using a high-speed digital interface, such as low voltage differential signaling (LVDS), asynchronous serial communication, such as a universal asynchronous receiver transmitter (UART), a low-delay network protocol, such as error synchronous serial communication or a controller area network (CAN), optical communication, or any of other various communication methods that are well known to one of ordinary skill in the art.

In addition, the exemplary embodiments may also be implemented through computer-readable code and/or instructions on a medium, e.g., a computer-readable medium, to control at least one processing element to implement any above-described embodiments. The medium may correspond to any medium or media which may serve as a storage and/or perform transmission of the computer-readable code.

The computer-readable code may be recorded and/or transferred on a medium in a variety of ways, and examples of the medium include recording media, such as magnetic storage media (e.g., ROM, floppy disks, hard disks, etc.) and optical recording media (e.g., compact disc read only memories (CD-ROMs) or digital versatile discs (DVDs)), and transmission media such as Internet transmission media. Thus, the medium may have a structure suitable for storing or carrying a signal or information, such as a device carrying a bitstream according to one or more exemplary embodiments. The medium may also be on a distributed network, so that the computer-readable code is stored and/or transferred on the medium and executed in a distributed fashion. Furthermore, the processing element may include a processor or a computer processor, and the processing element may be distributed and/or included in a single device.

The foregoing exemplary embodiments are examples and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

## Claims

1. A method of processing a magnetic resonance (MR) image of an object, the method comprising:
receiving a user input for displaying, on a screen, first MR images and second MR images that are obtained by performing an MR imaging (MRI) scan on the object at different times;
classifying the first MR images into a plurality of first series according to a protocol of the object;
displaying the plurality of first series in a first region of the screen;
classifying the second MR images into a plurality of second series respectively corresponding to the plurality of first series, the plurality of second series being arranged in a second order that is same as a first order of the plurality of first series; and
displaying the plurality of second series in a second region of the screen.

2. The method of claim 1, wherein the receiving comprises:
receiving a user input for displaying the first MR images on the screen; and
receiving a user input for displaying the second MR images on the screen in response to the receiving the user input for displaying the first MR images.

3. The method of claim 1 or 2, wherein the classifying the first MR images comprises arranging the plurality of first series in a matrix form, and
the classifying the second MR images comprises arranging the plurality of second series in a matrix form.

4. The method of claim 1, 2 or 3, wherein the first MR images and the second MR images are obtained by respectively performing MRI scans on different objects.

5. The method of any of claims 1-4, further comprising:
receiving a user input for selecting a first series from the displayed plurality of first series;
selecting the first series from the plurality of first series based on the user input for selecting the first series; and
determining whether a second series corresponding to the selected first series is included in the plurality of second series.

6. The method of claim 5, further comprising:
selecting the second series from the plurality of second series in response to the determining that the second series corresponding to the selected first series is included in the plurality of second series; and
displaying an enlarged version of the selected second series in the second region, preferably
further comprising displaying a blank image in an area of the second region corresponding to the selected first series in response to the determining that the second series corresponding to the selected first series is not included in the plurality of second series.

7. The method of any of claims 1-6, further comprising:
receiving a user input for changing the first order in which the plurality of first series is arranged;
changing the first order in which the plurality of first series is arranged, based on the user input for changing the first order in which the plurality of first series is arranged; and
changing the second order in which the plurality of second series is arranged to correspond to the first order in which the plurality of first series is arranged.

8. The method of any of claims 1-7, further comprising:
receiving a user input for searching for a first series among the plurality of first series and a second series among the plurality of second series;
searching for the first series among the plurality of first series and the second series among the plurality of second series based on the user input for searching for the first series and the second series;
displaying the first series found among the plurality of first series in the first region; and
displaying the second series found among the plurality of second series in the second region,
wherein preferably only the found first series and the found second series are displayed on the screen.

9. The method of any of claims 1-8, further comprising displaying, in a third region of the screen, one or more icons for executing one or more applications capable of analyzing the object, preferably further comprising:
receiving a user input for selecting a first series from the displayed plurality of first series;
displaying, on the selected first series, a mark distinguishing the selected first series from series unselected from the plurality of first series; and
displaying, on an icon for executing an application capable of analyzing the object by using the selected first series, among the one or more icons, a mark identical to the mark displayed on the selected first series, preferably further comprising:
selecting a second series corresponding to the selected first series from the plurality of second series; and
displaying, on the selected second series, a mark identical to the mark displayed on the selected first series.

10. The method of any of claims 1-9, further comprising:
receiving a user input for selecting a first series from the displayed plurality of first series; and
displaying first MR images included in the selected first series sequentially with respect to time, preferably further comprising:
selecting a second series corresponding to the selected first series from the plurality of second series; and
displaying second MR images included in the selected second series sequentially with respect to time.
preferably further comprising:
receiving a user input for displaying, on the screen, third MR images that are obtained by performing an MRI scan on the object;
classifying the third MR images into a plurality of third series respectively corresponding to the plurality of first series and the plurality of second series, the plurality of third series being arranged in a third order that is same as the first order of the plurality of first series and the second order of the plurality of second series, and the plurality of third series being arranged in a matrix form; and
displaying the plurality of third series in a third region of the screen.

11. A non-transitory computer-readable storage medium storing a program comprising instructions to cause a computer to perform the method of any of claims 1-10.

12. An apparatus for processing a magnetic resonance (MR) image of an object, the apparatus comprising:
an image processor configured to:
receive a user input for displaying, on a screen, first MR images and second MR images that are obtained by performing an MR imaging (MRI) scan on the object at different times;
classify the first MR images into a plurality of first series according to a protocol of the object; and
classify the second MR images into a plurality of second series respectively corresponding to the plurality of first series, the plurality of second series being arranged in a second order that is same as a first order of the plurality of first series; and
a display comprising the screen including a first region and a second region, the display being configured to:
display the plurality of first series in the first region; and
display the plurality of second series in the second region.

13. The apparatus of claim 12, wherein the image processor is further configured to:
arrange the plurality of first series in a matrix form; and
arrange the plurality of second series in a matrix form.

14. The apparatus of claim 11 or 12, wherein the image processor comprises a user input interface configured to receive the user input for displaying the first MR images and the second MR images.

15. The apparatus of claim 12, 13 or 14, wherein the user input interface is further configured to implement the method steps of any of claims 1-11.
